(19) Europäisches Patentamt
European Patent Office
Office européen des brevets

(11) **EP 3 569 272 B1**

(12) **EUROPÄISCHE PATENTSCHRIFT**

(45) Veröffentlichungstag und Bekanntmachung des Hinweises auf die Patenterteilung:
**23.07.2025 Patentblatt 2025/30**

(21) Anmeldenummer: **19172257.8**

(22) Anmeldetag: **02.05.2019**

(51) Internationale Patentklassifikation (IPC):
**A61M 5/31** *(2006.01)* **A61M 39/20** *(2006.01)*

(52) Gemeinsame Patentklassifikation (CPC):
**A61M 5/31; A61M 39/20;** A61M 5/3134;
A61M 2005/3104; A61M 2005/3118

(54) **VERSCHLUSS FÜR EINEN BEHÄLTER FÜR PHARMAZEUTISCHE PRÄPARATE UND MIT DIESEM VERSCHLUSS VERSEHENER BEHÄLTER**

CLOSURE FOR A CONTAINER FOR PHARMACEUTICAL PREPARATIONS AND CONTAINER THAT HAS BEEN CLOSED

FERMENTURE DE RECIPIENT POUR PREPARATIONS PHARMACEUTIQUES ET DE RECIPIENTS FERMES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.05.2018 DE 102018111449**
**14.05.2018 DE 202018102680 U**

(43) Veröffentlichungstag der Anmeldung:
**20.11.2019 Patentblatt 2019/47**

(73) Patentinhaber: **SCHOTT Pharma Schweiz AG**
**9000 St. Gallen (CH)**

(72) Erfinder: **KÜCÜK, Mustafa**
**9522 Staad (CH)**

(74) Vertreter: **Blumbach · Zinngrebe Patentanwälte PartG mbB**
**Alexandrastraße 5**
**65187 Wiesbaden (DE)**

(56) Entgegenhaltungen:
**EP-A1- 1 410 819     EP-A1- 3 015 130**
**WO-A1-2012/116791     WO-A1-2013/149821**
**US-B1- 9 821 152**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft allgemein einen Verschluss für einen Behälter für pharmazeutische Präparate und einen mit diesem Verschluss versehenen Behälter für pharmazeutische Präparate, insbesondere eine Spritze. Behälter für pharmazeutische Präparate, insbesondere Spritzen, leisten einen wesentlichen Beitrag im Rahmen der der Verabreichung von pharmazeutischen Präparaten bzw. Medikamenten. Diese im allgemeinen zylindrisch geformten Behälter umfassen eine Kammer zum Aufnehmen des Präparates, welche beidseitig von einem proximalen und einem distalen Ende begrenzt wird. Ein Kolben kann in das proximale Ende eingesetzt werden, um in einer gleitenden Bewegung das Präparat in distale Richtung und durch eine Öffnung an dem distalen Ende der Spritze zu drücken oder in einer umgekehrten, proximalen Bewegung ein Präparat durch diese Öffnung in die Spritze einziehen. Die Öffnung am distalen Ende wird dabei häufig durch eine konische Spitze gebildet. Zum Verschließen der distalen Öffnung des Behälters sind Verschlüsse oder Verschlusssysteme seit längerer Zeit bekannt. So beschreiben etwa die Druckschriften US 5,624,402 oder auch US 6,196,998 B1 derartige Verschlüsse zum Verschließen einer distalen Öffnung einer Spritze. Weitere Verschlusssysteme sind etwa in der Druckschrift EP 1 600 190 B1 beschrieben.

Die Druckschrift WO 2012/116791 A1 beschreibt einen weiteren Verschluss für eine Spritze mit einem Dichtelement, wobei der Verschluss über Stege und Vorsprünge verfügt, welche in einen Flansch einer Spritze eingreifen können. Die Druckschrift EP 1 419 819 A1 beschreibt einen Originalitätsverschluss für eine Spritze. Die Druckschrift WO 2013/149821 A1 beschreibt ein Verschlusssystem für einen Applikationseinrichtungs-Ansatz. Die Druckschrift EP 3 015 130 A1 beschreibt eine Schutzkappe für Strömungsanschlussstücke für medizinische Applikationen. Die Druckschrift US 9 821 152 B1 schließlich beschreibt eine weitere Verschlussbaugruppe für Spritzen.

Die bekannten Verschlüsse bzw. Verschlusssysteme weisen mehrere Nachteile auf.

So sind die bekannten Verschlüsse häufig mehrteilig ausgebildet und weisen ein inneres Element aus einem flexiblen Material zum Abdichten sowie ein äußeres Element auf, welches die Verbindung zu dem Behälter schafft. Hierfür ist häufig eine Schraubverbindung vorgesehen. Das Aufschrauben dieser mehrteiligen Verschlüsse gestaltet sich schwierig, wenn, wie aus dem Stand der Technik bekannt, das innere Element während des Fügens zuerst mit dem Behälter in Kontakt gelangt. Hierbei kann es sehr leicht zu einem Verkippen oder Verkanten zu Beginn Fügeprozesses kommen, was den geforderten kurzen Prozesszeiten zur Montage des Verschlusses entgegensteht.

Zudem ist das Innenelement häufig aus einem elastomeren Material gefertigt, welches herstellbedingt kontaminiert sein kann, beispielsweise mit Silikonöl. Die Verschlüsse werden im Allgemeinen als Schüttgut gelagert und transportiert. Wenn nun dieses teilweise kontaminierte elastomere Material des Innenelements in Kontakt mit einem Außengewinde eines anderen Verschlusses oder eines Behälters gelangt, so kann eine Kontaminierung dieses Bauteiles erfolgen, was für die Montage des Verschlusses und auch für das spätere Öffnen kritisch sein kann, da vorgegebene Kräfte und Momente unter Umständen nicht mehr sicher eingehalten werden können, so dass die Zuverlässigkeit der Verbindung und damit des gesamten Produktes nicht mehr gegeben sein kann.

Wünschenswert wären demnach ein Verschluss und ein Verfahren zur Montage dieses Verschlusses auf dem Behälter, welche eine kontaminationsfreie und sichere Montage mit kurzen Prozesszeiten ermöglichen.

Weitere Nachteile liegen in der Präzision und der Charakteristik des Anzieh- und/oder Öffnungsdrehmoments zur Montage bzw. zum Lösen des Verschlusses auf dem Behälter. Bekannte breite Gewindegänge des Verschlusses können insbesondere beim Lösen, also beim Öffnen des Behälters, zu einem konstant hohen Öffnungsdrehmoment führen, welches der Benutzer überwinden muss.

Wünschenswert wären es daher, einen Verschluss mit einem Öffnungsdrehmoment bereitzustellen, welcher es erlaubt, einen vorgegebenen Schwellwert für das Öffnen des Verschlusses präzise bereit zu stellen, gleichzeitig aber das anschließende Lösen vereinfacht.

Weitere Probleme liegen in der hohen Präzision der geforderten Abstände der zum Abdichten erforderlichen Komponenten in montierter Position zueinander. Von hoher Bedeutung ist hierbei der axiale Abstand des zur Abdichtung vorgesehenen Elements zur zu verschließenden Öffnung des Behälters. Die gegebenen Form- und Lagetoleranzen können dazu führen, dass eine geforderte Mindestpressung zur Abdichtung nicht eingehalten werden kann, wodurch die Zuverlässigkeit des Produktes in Frage gestellt werden kann.

Wünschenswert wären es daher, einen Verschluss bereitzustellen, welcher es erlaubt, die geforderte Mindestpressung zur Abdichtung der Öffnung des Behälters sicher zu gewährleisten.

Weitere Nachteile liegen in der Farbstabilität des Materials des Verschlusses, welches häufig Kunststoff umfasst. Diese Farbstabilität ist insofern von Bedeutung, als dass der Verschluss und/oder der mit einem Verschluss versehene Behälter einer Behandlung zur Sterilisation unterzogen werden kann, welche zu Farbveränderungen oder sonstigen Eigenschaftsänderungen der beteiligten Kunststoffe führen kann.

Wünschenswert wären es daher, einen Verschluss bereitzustellen, welcher es erlaubt, die gängigen Methoden zur Sterilisation anzuwenden, ohne dass es zu einer sichtbaren Farbveränderung oder sonstigen Veränderung des Verschlusses kommt.

Dieser Aufgabe haben sich die Erfinder angenommen und einen Verschluss für einen zylinderförmigen Behälter für pharmazeutische Präparate, insbesondere eine Spritze, sowie einen mit diesem Verschluss versehenen Behälter für pharmazeutische Präparate, insbesondere eine Spritze, nach einem der unabhängigen Ansprüche zur Verfügung gestellt.

Der erfindungsgemäße Verschluss vermeidet die oben genannten Nachteile und verfügt daher über mehrere konstruktive Verbesserungen im Vergleich zu bekannten Verschlüssen oder Verschlusssystemen. Bevorzugte Ausführungsformen und Weiterbildungen der Erfindung sind den jeweiligen Unteransprüchen zu entnehmen.

Zylinderförmige Behälter für pharmazeutische Präparate sind in der Regel langgestreckt und verfügen über eine zentrale Kammer sowie ein sich auf der einen Seite der Kammer anschließendes proximales Ende, in das etwa ein Kolben eingesetzt werden kann, und ein gegenüberliegend angeordnetes distales Ende, welches eine konisch zulaufende Spitze umfassen kann. Die Mittenachse des Behälters stellt die Längsachse der nachfolgend beschriebenen Anordnungen dar.

Das distale Ende ist im Allgemeinen von langgestreckter, zum distalen Ende hin konisch zulaufender Form ausgebildet und weist eine Öffnung in Form eines engen Durchgangs auf, durch den beispielsweise flüssige pharmazeutische Präparate aus der Kammer ausgetrieben werden können, sofern eine entsprechende Kolbenbewegung erfolgt. Umgekehrt können auch flüssige Präparate eingezogen werden, sofern der Kolben in die entgegengesetzte Richtung, also in Richtung proximales Ende, bewegt wird. Im pharmazeutischen Bereich sind diese zylinderförmigen Behälter als Spritze oder Kanüle bekannt und können aus Glas oder Kunststoff gefertigt sein. Der erfindungsgemäße Verschluss ist grundsätzlich sowohl für Behälter aus Glas als auch aus Kunststoff geeignet. Die pharmazeutischen Präparate können Flüssigkeiten, Gase, Feststoffe oder Gemische hieraus umfassen.

Zur Befestigung etwa einer Nadelkanüle an dem distalen Ende des Behälters hat sich im medizinischen Bereich eine als Luer-Lock bezeichnete Einrichtung etabliert. Bei Behältern aus Kunststoff kann diese Luer-Lock-Befestigung einstückig mit dem Behälter ausgeführt sein, etwa als Manschette oder Kragen, und die Spitze des Behälters zumindest teilweise umgeben. Diese Luer-Lock-Befestigung kann eine Manschette mit einem Innengewinde am distalen Ende des Behälters umfassen.

Zur Abdichtung der Öffnung am distalen Ende des Behälters oder zum Verschließen des Durchgangs der Spitze ist ein Verschluss erforderlich, etwa, um Verunreinigungen eingefüllter Präparate zu vermeiden oder einem unbeabsichtigten Verlust des Präparates entgegenzuwirken. Unter dem Begriff "Abdichten" oder "Verschließen" wird nachfolgend ein druck- und/oder fluiddichtes Verschließen der Öffnung des distalen Endes des Behälters verstanden.

Gegenstand der vorliegenden Erfindung ist demnach ein Verschluss zum lösbaren Verschließen einer Öffnung am distalen Ende eines zylinderförmigen Behälters für pharmazeutische Präparate, insbesondere einer Spritze, vorzugsweise mittels einer Luer-Lock-Befestigung.

Der erfindungsgemäße Verschluss ist besonders gut geeignet zum Verschließen der Öffnung eines distalen Endes des Behälters, welches als Spitze ausgebildet ist. Der Behälter ist vorzugsweise aus Kunststoff gefertigt und umfasst eine Manschette mit einer Luer-Lock-Befestigung mit einem Innengewinde.

Der vorgesehene Verschluss umfasst:

eine zylindrische Verschlusskappe mit einem zylindrischen Hohlraum zur Aufnahme und Halterung eines Dichtelements, wobei

die Verschlusskappe einen Montageabschnitt umfasst, welcher in montierter Position zu dem Behälter hinweist,

ein Dichtelement mit einem Dichtabschnitt, welcher zumindest teilweise innerhalb des Montageabschnittes angeordnet ist, und wobei

die Außenkante des Dichtelements gegenüber der Außenkante des Montageabschnittes einen Rückversatz aufweist.

Die Außenkante des Dichtelements meint hier die in montierter Position zum Behälter hinweisende äußere Kante des Dichtelements; die Außenkante des Montageabschnittes ebenfalls die in montierter Position zum Behälter hinweisende äußere Kante des Verschlusses.

Die Verschlusskappe kann ein- oder mehrteilig ausgeführt sein und in einer günstigen Ausführungsform einen Montageabschnitt, der zur Verbindung mit dem Behälter ausgelegt ist und somit in montierter Position zum Behälter hinweist, und einen gegenüberliegend angeordneten Betätigungsabschnitt umfassen, der einem Benutzer das Greifen des Verschlusses ermöglicht.

Erfindungsgemäß umfasst der Montageabschnitt ein Außengewinde, welches in montierter Position in die Luer-Lock-Befestigung des Behälters eingreift und auf diese Weise eine lösbare Verbindung schaffen kann. In montierter Position sind Verschlusskappe und Behälter koaxial zur Längsachse angeordnet. Der erfindungsgemäße Verschluss mit in der Verschlusskappe eingesetztem Dichtelement ist besonders geeignet für die Montage an einem Behälter mit einer Luer-Lock-Befestigung, welche ein Innengewinde zur Aufnahme eines passgenau gegengleichen Außengewindes umfasst.

Der Betätigungsabschnitt kann vorzugsweise mit Außenrippen zur besseren Handhabung durch den Benutzer ausgestattet sein. Der zylindrische Hohlraum der Verschlusskappe ist durchgehend ausgebildet zum einfachen Setzen und Haltern eines vorzugsweise einstückigen Dichtelements, welches ebenfalls zylinderförmig ausgebildet sein kann und eine Außengeometrie passgenau gegengleich zu der Innengeometrie des zylindrischen Hohlraumes der Verschlusskappe aufweisen kann. Das Dichtelement ist vorzugsweise aus einem elastomeren Material gefertigt.

Der erfindungsgemäße Rückversatz ermöglicht es in verblüffend einfacher Weise, den Montageprozess deutlich zu verbessern und die Prozesssicherheit zu erhöhen, da ein Verkippen und Verkanten während des Aufsetzens des Verschlusses auf den Behälter wirksam verhindert werden kann. Dies ist dem Umstand geschuldet, dass während des Fügens zunächst eine Zentrierung des Verschlusses über den Montageabschnitt erfolgt, bevor das Dichtelement in Kontakt mit dem Behälter, insbesondere mit der Spitze des Behälters, gelangt. Gerade bei vollautomatisierten Montageprozessen und den geforderten hohen Stückzahlen ist eine Erhöhung der Montagesicherheit von großer Bedeutung für die Effizienz der Herstellung.

Der Rückversatz des Dichtelements gegenüber der Außenkante des Montageabschnittes der Verschlusskappe, in anderen Worten also ein gegenüber der Außenkante vertieftes Dichtelement auf der Montageseite des Verschlusses, erhöht also die Prozesssicherheit, da eine Zentrierung des Verschlusses beim Einschraubvorgang bereits vor einem Kontakt des Dichtelements mit dem Behälter erfolgt. Am Markt existierende Verschlüsse weisen dagegen ein Dichtelement auf, welches aus der Verschlusskappe in Montagerichtung hervorsteht. Bei einer Montage erfolgt dann der Erstkontakt zwischen Dichtelement und Behälter, was eine prozesstechnisch gewünschte Zentrierung erschwert bzw. sogar unmöglich macht.

Vorliegend erfolgt der erste Kontakt zur Montage des Dichtelements mit der Spitze des Behälters erst nach einer Zentrierung von Verschluss und Behälter, so dass ein rein axiales Aufschieben des Dichtelements möglich ist, was weniger fehleranfällig ist.

Gleichzeitig führt der Rückversatz des Dichtelements auch zur einer Verringerung der Kontamination anderer Verschlüsse oder auch des Behälters, etwa durch Fertigungsrückstände, welche sich auf der Oberfläche des Dichtelements anreichern können. Dies können beispielsweise in der Fertigung der Dichtelemente verwendete Flüssigkeiten, etwa Silikonöl, sein, welches sich außen auf dem Dichtelement ansammeln kann. Gerade bei Dichtelementen, welche aus einem elastomeren Material gefertigt sind, ist dies häufig der Fall.

Die Verschlüsse mit dem Dichtelement werden häufig als Schüttgut in mehrere tausend Stück in einer Verpackung, etwa einem Beutel gelagert, transportiert und zugeliefert. Wenn nun das Dichtelement aus dem Verschluss hervorsteht, kann dieses, sofern es verunreinigt ist, durch Kontakt auch andere Verschlüsse im Schüttgut kontaminieren. So können beispielsweise in der Verpackung silikonisierte, also mit Silikonöl verunreinigte Dichtelemente, andere, nicht silikonisierte Verschlüsse berühren. Durch den Kontakt kann ein nicht silikonisierter Verschluss mit Silikonöl kontaminiert werden.

Wenn hierbei beispielsweise ein Außengewinde einer anderen Verschlusskappe mit Silikonöl kontaminiert wird, so ist diese Kontamination nachteilig beim Transport oder auch bei Folgeprozessen, etwa dem Vereinzeln, dem Greifen, oder dem Setzen des Verschlusses zur Montage auf den Behälter an der entsprechenden Maschine. Durch den Rückversatz des Dichtelements kann dieser unerwünschten Kontamination entgegengewirkt werden.

Auch produktseitig bringt eine Kontaminierung des Außengewindes der Verschlusskappe Nachteile mit sich, etwa hinsichtlich des zur Montage geforderten Anziehdrehmoments und des späteren zum Lösen erforderlichen Löse- oder Öffnungsdrehmoments. Kommt beispielsweise ein kontaminiertes Dichtelement in Kontakt mit einem Außengewinde, so kann das Anziehdrehmoment deutlich herabgesetzt werden oder es kann starken Schwankungen unterworfen sein.

In Bezug auf den Behälter kann durch eine Reduzierung der Gefahr einer Kontamination die Zuverlässigkeit des mit dem Verschluss verschlossenen Behälters gesteigert werden, wenn sicher ausgeschlossen werden kann, dass sich etwa im Zuge des Herstell- und/oder Montageprozesses Fertigungsrückstände des Dichtelements auf einem Gewinde ablagern können.

So konnte in Versuchen beispielsweise nachgewiesen werden, dass das Anziehdrehmoment und das Öffnungsdrehmoment infolge einer Kontamination des Außengewindes stark zu streuen beginnen. Dies bedeutet, dass ein vorgegebenes Moment nicht mehr eingehalten werden kann. Die gemessene Streuung des Anziehdrehmoments bei kontaminierten Verschlüssen kann mehr als 0,5 N*cm, mehr als 1,0 N*cm oder sogar mehr als 1,5 N*cm betragen. Die Streuung der Öffnungsdrehmomente bei kontaminierten Verschlüssen kann zwischen 2 bis 16 N*cm, zwischen 2 bis 18 N*cm oder sogar zwischen 2 bis 20 N*cm betragen. Derartig starke Streuungen sind nachteilig für einen Benutzer.

Von Vorteil ist daher auch auf der gegenüberliegenden Seite der Verschlusskappe ein derartiger Rückversatz des Dichtelements gegenüber der Außenkante des Betätigungsabschnitts vorgesehen, um die Kontaminationsgefahr zu verringern.

Erfindungsgemäß wird daher nicht nur ein Beitrag zur Erhöhung der Prozesssicherheit, sondern auch zur Produktzuverlässigkeit der Behälter für pharmazeutische Präparate geleistet. Es kann ein Verschluss bereitgestellt werden, bei welchem das Anziehdrehmoment und/oder das Öffnungsdrehmoment eine geringe Streuung von +/- 1,0 N*cm, bevorzugt von +/- 0,5 N*cm zum geforderten oder gewünschten Drehmoment aufweisen.

Dies wird dadurch erreicht, dass die Länge des Dichtelementes so ausgelegt wird, dass die potentiell kontaminierten Dichtelemente andere Verschlusskappen nicht berühren können. Die Länge des Dichtelements entlang der Längsachse ist damit von Vorteil geringer als die Länge der Verschlusskappe. Im Verhältnis zur Länge der Verschlusskappe beträgt die Länge des Dichtelements in einer bevorzugten Ausführungsform höchstens 95 %, bevorzugt höchstens 90 % und besonders bevorzugt höchstens 85 %.

Das Montage- und Demontageverhalten der Verschlusskappe wird zu einem wesentlichen Anteil durch die Ausbildung des Außengewindes der Verschlusskappe bestimmt. Sofern der Behälter über eine Luer-Lock-Befestigung verfügt, kann durch eine besondere Gestaltung des Außengewindes der Verschlusskappe eine einfachere und präzisere Montage ermöglicht werden, welche ebenfalls die Prozesssicherheit sowie auch die Produktzuverlässigkeit erhöhen kann.

Hierzu kann das Außengewinde der Verschlusskappe gemäß der gängigen Norm ISO 594-2 ausgebildet sein, wobei erfindungsgemäß aber eine besondere Ausbildung des Gewindes, vorzugsweise eine andere Form der Gewindeflanke, vorgesehen ist, was zu einem anderen Eingriff des Außengewindes in das Innengewinde eines Luerlock-Anschlusses führt. Die Erfindung sieht in einer bevorzugten Ausführungsform vor, die Gewindeflanke schmaler und/oder mit steilerer Flanke auszubilden, was bei gleicher Steigung zu einem größeren Abstand der Gewindeflanken zueinander führt und damit zu einem geringen axialen Spiel der erst teilweise eingeschraubten Verschlusskappe in einen Luerlock-Anschluss.

Das Gewinde ist dabei vorzugsweise als Trapezgewinde ausgebildet, bevorzugt mit einer Außenbzw. Kammbreite ("thread crest width") von etwa 0,6 mm und einer Kernbreite ("maximum width of base") von etwa 1,2 mm. Dies führt zunächst zu einem einfacheren Aufschrauben des Verschlusses auf den Behälter, da die wirkenden Reibungskräfte geringer sind. Andere konstruktive Kenngrößen des Gewindes bleiben dabei vorzugsweise gleich, etwa die Gewindesteigung.

Eine vorteilhafte Gewindesteigung im Sinne der Erfindung beträgt zwischen 3 und 5,5 mm, bevorzugt zwischen 3,5 und 5,25 mm und besonders bevorzugt zwischen 4 und 5 mm.

Dies ermöglicht es ferner, auf der Gewindeflanke an zumindest einer bestimmten Position eine Erhebung, vorzugsweise in punktförmiger Form, vorzusehen, welche etwa zwischen 0,5 und 1,5 mm gegenüber der Flanke hervorsteht. Vorzugsweise sind wenigstens zwei an gegenüberliegenden Positionen der Gewindeflanke angeordnete Erhebungen vorgesehen. Zwei gegenüberliegend angeordnete Erhebungen können damit ein sogenanntes Erhebungspaar ausbilden. Ein derartiges Erhebungspaar ist vorzugsweise derart angeordnet, dass in montierter Position die Erhebungspunkte dann an der Gewindeflanke der Luer-Lock-Befestigung anliegen und vorzugsweise hierbei leicht gestaucht bzw. deformiert werden. Damit geht eine zusätzliche Kraftkomponente in die Verbindung von Verschluss und Behälter ein. Gegenüber einer einzelnen Erhebung bietet ein Erhebungspaar den Vorteil, dass diese zusätzliche Kraftkomponente nicht einseitig wirkt.

Diese Kraftkomponente wirkt gleichermaßen auch bei dem Öffnen des Verschlusses und ermöglicht es damit, einen vorgegebenen Schwellwert für das Öffnen des Verschlusses präzise bereit zu stellen. Auf diese Weise kann eine bessere, für den Benutzer einfachere Charakteristik für das Öffnungsdrehmoment zum Lösen des Verschlusses von dem Behälter bereitgestellt werden.

Für eine gleichmäßige Krafteinbringung ist es vorteilhaft, mehr als ein Erhebungspaar vorzusehen, bevorzugt zwei, besonders bevorzugt vier Erhebungspaare. Hierdurch kann ein Beitrag für einen gleichmäßigen und festen Sitz des Verschlusses auf dem Behälter gesorgt werden. Auf diese Weise wird weiter begünstigt, das für die Montage und/oder das Lösen erforderliche Drehmoment präzise einzustellen, sowie auch einen vorbestimmten Kräfteverlauf während der Drehbewegung sicherzustellen.

Generell ist bei der Montage eines Verschlusses auf Behälter, welche für pharmazeutische Präparate genutzt werden, auf die präzise Einhaltung vorgeschriebener Kräfte zu achten, da ein Über- oder Unterschreiten sich rasch ungünstig auf die Produktsicherheit auswirken kann.

Mittels des erfindungsgemäß ausgebildeten Außengewindes der Verschlusskappe ist es möglich, ein sehr enges Montagedrehmoment von beispielsweise 18.0 N*cm bei einer Toleranz von +/- 1,0 N*cm, bevorzugt von +/- 0,5 N*cm einzuhalten. Dies ist wichtig, da ein Anziehdrehmoment von mehr als etwa 20 N*cm bei Luer-Lock-Befestigungen, insbesondere aus Kunststoff, zu einer Mikrorissbildung führen kann. Dies kann zu einem Versagen des Behälters und/oder des Verschlusses führen, wodurch die Produktzuverlässigkeit nicht mehr gegeben ist. Ist das Anziehdrehmoment kleiner als 16 N*cm, so kann die Dichtigkeit bei Spitzen mit einem geringer Luer-Lock-Überstand kaum noch gewährleistet werden.

Die besondere Gestaltung des Gewindes führt weiterhin dazu, dass beim Öffnen des Verschlusses für das Lösen zunächst eine höhere Kraftkomponente aufgewendet werden muss, wonach sich diese Kraftkomponenten konstant reduziert. Dies erleichtert das Öffnen des Behälters und das Lösen des Verschlusses für den Benutzer. Das durch den Benutzer aufzubringende Drehmoment richtet sich demnach nach dem Öffnungswinkel, also der Orientierung von Verschluss zu Behälter. Nach Überwinden eines Anfangswiderstandes zu Beginn des Lösens, welches sich infolge der zusätzlich wirkenden Kraftkomponente aufgrund der Stauchung der Erhebungspunkte einstellt, sinkt die einwirkende Kraftkomponente aufgrund der schmaleren Gewindegänge, welche nach dem Lösen den Erhebungspunkten mehr Freiraum bieten, so dass die Erhebungen weniger oder vorzugsweise nicht mehr gestaucht werden. Auf diese Weise geht das zum weiteren Lösen aufzubringende Drehmoment zurück. Damit kann ein präzises Drehmoment für das Öffnen, beispielsweise von 16 N*cm mit einer Genauigkeit von +/- 0,5 N*cm, eingestellt werden.

Somit unterscheidet sich das Löseverhalten des erfindungsgemäßen Verschlusses auch von bekannten Verschlüssen, bei welchen die zu Beginn des Lösens aufzuwendende Kraftkomponenten während des Lösens des Verschlusses kaum sinkt. Dies ist dem Umstand geschuldet, dass bekannte Verschlüsse häufig eine Aufwölbung des Gewindegangs für eine zusätzliche Kraftkomponente vorsehen, welche dann aber während des gesamten Lösens wirkt aufgrund des breiteren Gewindeganges. Für den Benutzer ist diese Charakteristik des zum Lösen aufzubringenden Drehmoments eher ungünstig, da für nahezu das gesamte Öffnen ein höheres Moment aufgebracht werden muss.

Erfindungsgemäß wird die Montage des Verschlusses weiterhin dadurch vereinfacht, dass der Gewindegang zum einen wie oben ausgeführt schmaler ist, was die mögliche Winkelorientierung, also die winkelrichtige Ausrichtung von Verschluss und Behälter zueinander zu Beginn der Montage, vergrößert und damit erleichtert.

Um diesen Toleranzbereich der geforderten Winkelorientierung noch stärker zu erweitern, ist in einer bevorzugten Ausführungsform eine Aufwölbung des neben dem Gewindeeingang liegenden Abschnittes des Gewindegangs zur Vergrößerung des Freiraumes von der Außenkante bis zu dem ersten Gewindegang vorgesehen, wodurch sich der Toleranzbereich der Winkelorientierung nochmals vergrößern lässt. Diese Aufwölbung stellt damit einen leichten axialen Versatz des Gewindegangs in dem besagten Abschnitt dar. Aufgrund des schmaleren Gewindegangs kann diese Aufwölbung daher von Vorteil auch derart gestaltet werden, dass keine zusätzliche Kraftkomponente auf die Verbindung einwirkt.

Ein enger Toleranzbereich für das winkelrichtige Aufstecken und Einschrauben des Verschlusses auf den Behälter ist damit nicht erforderlich. Vielmehr ist ein größerer Winkelbereich gegeben, innerhalb dessen die Montage erfolgen kann. Während bei bekannten Verschlüssen dieser Toleranzbereich in Bezug auf das winkelrichtige Montieren, also ein Abweichen von der idealen Orientierung bei dem Fügen, von weniger als +/- 3° gegeben ist, so ermöglicht die erfindungsgemäße Gestaltung des Gewindeeinstiegs, diese Winkeltoleranz auf einen Bereich von +/- 10° oder sogar darüber hinaus zu erweitern. Je nach konkreter Gestaltung kann diese erfindungsgemäße Winkeltoleranz in einem Bereich zwischen +/- 5° und +/- 15°, bevorzugt zwischen +/- 8° und +/- 12° liegen. Dies führt zu einem deutlich günstigeren Montageverhalten, da der Aufwand für die exakte Orientierung des Verschlusses in Bezug auf den Behälter reduziert werden kann.

In einer ebenfalls bevorzugten Ausführungsform kann der Gewindeeingang zusätzlich eine Fase umfassen bzw. die Gewindeeinführung als Fase ausgebildet sein. Während bekannte Verschlüsse ein Außengewinde mit einer stumpfen Gewindeeinführung aufweisen, welche sehr rasch zu einem Verkanten während des Beginns der Montage führen kann, so erleichtert diese Einführschräge bzw. Fase an der Gewindeeinführung die Montage erheblich.

Die Zuverlässigkeit des mit dem erfindungsgemäßen Verschluss versehenen Behälters pharmazeutische Präparate wird weiterhin durch die Passgenauigkeit derjenigen Komponenten, welche die Dichtheit der Öffnung des Behälters bewirken, beeinflusst. Von entscheidender Bedeutung ist hier der axiale Abstand zwischen einem radial nach außen hervorstehenden Anschlag der Verschlusskappe, welcher in montierter Position an dem Behälter anliegt, insbesondere an dem Kragen der Luer-Lock-Befestigung, und der Innenkante eines Dichtabschnitts des Dichtelements, welcher die Abdichtung der Öffnung der Spitze bewirkt. Der Dichtabschnitt des Dichtelements liegt dabei in montierter Position eng an der Öffnung, insbesondere an der Spitze, an und/oder umschließt die Öffnung, wobei auf eine Mindestpressung geachtet werden muss, welche über die Form- und Lagetoleranzen der beteiligten Komponenten eingestellt werden kann.

Zur axialen Positionierung des Dichtelements in der Öffnung der Verschlusskappe ist diese vorzugsweise mit einer radial nach innen vorspringenden, umlaufenden Schulter ausgebildet. Das Dichtelement weist von Vorteil auf seiner Mantelfläche eine ebenfalls umlaufende Ausnehmung auf, welche vorzugsweise derart positioniert ist, dass in montierter Position die Schulter der Verschlusskappe in die Ausnehmung des Dichtelements eingreifen und eine Arretierung durch diesen axialen Anschlag bewirken kann. Auf diese Weise kann eine präzise Positionierung des Dichtelements und damit der Innenkante des Dichtelements, welche die Öffnung des Behälters abdichtet, in Bezug auf den Behälter in montierter Position erreicht werden.

Für gängige Behälter, insbesondere für gängige Spritzen, wird für den axialen Abstand zwischen dem nach außen hervorstehenden Anschlag der Verschlusskappe, auch als äußerer Anschlag bezeichnet, und dem durch die Schulter definierten Anschlag innerhalb der Öffnung der Verschlusskappe, auch als innerer Anschlag bezeichnet, ein Montagemaß von 2,85 mm mit einer sehr geringen Toleranz gefordert. Der innere Anschlag wirkt dabei in eine umgekehrte Richtung in Bezug auf die zur Abdichtung aufzubringenden Mindestpressung des Dichtelements und bestimmt somit die auf die Öffnung der Spitze wirkende Dichtkraft.

Demnach ist also für die Ausgestaltung des Verschlusses das Einhalten einer sehr engen Toleranz für diesen inneren und äußeren Anschlag von hoher Bedeutung. Erfindungsgemäß wird das Einhalten dieser Toleranz durch verschiedene konstruktive Maßnahmen gelöst.

Zum einen ist in einer bevorzugten Ausführungsform die Verschlusskappe derart ausgelegt, dass eine dünne und vor allen Dingen gleichmäßige Materialdicke der Seitenwandung über die Länge der Verschlusskappe gegeben ist. Vorzugsweise liegt die Wandstärke der Verschlusskappe, also die Dicke der Wandung, an der dicksten Stelle bei höchstens 2,5 mm, bevorzugt höchstens 2,0 mm. Dabei schwankt die Dicke der Wandung auf die gesamte Länge der Verschlusskappe gesehen um nicht mehr als 0,5 mm, bevorzugt um nicht mehr als 0,3 mm.

Die Einhaltung dieses Maßes ist nicht trivial. Die Verschlusskappen werden im Allgemeinen aus thermoplastischen Kunststoffen im Spritzgussverfahren hergestellt. Dies führt zu höheren Temperaturen in der Urformung, was nach Abkühlen zu Maßabweichungen der Verschlusskappe führen kann. Eine konstante Wanddicke führt zu einem homogenen Abkühlen und reduziert damit möglichen Verzug.

Ferner wird erfindungsgemäß das Spritzgussverfahren dahingehend optimiert, dass ein Einspritzpunkt gewählt wird, welcher im Bereich des Betätigungsabschnitts liegt. Besonders günstig liegt der Einspritzpunkt auf einer Position außenseitig auf der Mantelfläche des Betätigungsabschnitts axial beabstandet von einer Außenrippe. Hierdurch kann zudem einer möglichen Verletzungsgefahr für einen Benutzer entgegengewirkt werden, da durch den Anguss scharfe Kanten entstehen können. Nicht bevorzugt ist dagegen ein Einspritzpunkt auf einer der Außenrippen, da dies zu einem Verzug der dünnwandigen Verschlusskappe führen kann.

Das erfindungsgemäße Verfahren ermöglicht es, die Verschlusskappe im Spritzgussverfahren in sehr hohen Stückzahlen mit einer sehr engen Maßtoleranz zu fertigen, was zu einem Teil der dünnen und gleichmäßigen Wanddicke sowie der optimierten Position des Einspritzpunktes geschuldet ist.

Um die oben genannte enge Toleranz weiterhin einhalten zu können, ist es ferner hilfreich, dass das Dichtelement präzise in der Verschlusskappe gehalten wird. Insbesondere sind unbeabsichtigte Setzbewegungen des Dichtelements in axialer Richtung relativ zu der Verschlusskappe möglichst auszuschließen, welche etwa infolge des Setzens und/oder der Montage auftreten können.

Die drehsichere Halterung des Dichtelements in der durchgehenden Öffnung der Verschlusskappe kann über zumindest zwei längliche, in ihrer Hauptausrichtung axial angeordnete und radial nach Innen hervorstehende Innenrippen auf der Innenwandung der Öffnung, vorzugsweise im Bereich des Betätigungsabschnitts, erfolgen. Typischerweise sind etwa 6 bis 10 derartige Innenrippen, häufig auch 8 regelmäßig angeordnete Innenrippen vorgesehen. Diese Innenrippen können für eine gleichmäßige Pressung günstigerweise in gleichmäßigen Abständen über den Innenumfang verteilt angeordnet sein. Diese Innenrippen dienen also dazu, einer Verdrehung des Dichtelements in der Verschlusskappe, etwa bei der Montage oder beim Öffnen, entgegenzuwirken, und stellen damit eine Verdrehsicherung dar.

Aufgrund der Herstellung der Verschlusskappe im Spritzgussverfahren können diese Innenrippen eine Entformungsschräge in Längsrichtung, also in axialer Richtung, aufweisen. Jedoch entsteht durch diese Neigung der Innenrippen eine axial auf das Dichtelement wirkende Kraftkomponente, welche eine axiale Bewegung des Dichtelements aus der vorgesehenen Position heraus zur Folge haben kann. Trotz des inneren Anschlags kann diese Bewegung kann zu einem ungewollten axialen Versatz des Dichtelements von 0,3 mm oder sogar mehr gegenüber der vorbestimmten Setzhöhe führen.

Dieser Versatz beeinflusst damit den vorstehend beschriebenen axialen Abstand zwischen dem Anschlag der Verschlusskappe und der Innenkante des Dichtabschnitts des Dichtelements, so dass das vorgegebene Maß nicht immer sicher eingehalten werden kann. Dies kann auch dazu führen, dass die Mindestpressung zwischen Dichtelement und Spitze des Behälters nicht erreicht wird, so dass die geforderte Dichtigkeit unter Umständen nicht eingehalten werden und damit die geforderte Zuverlässigkeit des Behälters nicht gegeben sein kann. Bei einer Verwendung von Schraubverbindungen für pharmazeutische Verpackungen gilt beispielsweise eine Mindestanforderung, wonach die Verbindung wenigstens einem Druck von 3 bar für eine Dauer von 30 Sekunden widerstehen muss.

Zum anderen kann unter Umständen das geforderte Öffnungsdrehmoment, welches etwa für Schraubverschlüsse im pharmazeutischen Bereich bei wenigstens 2 N*cm liegt, nicht eingehalten werden, wenn der geforderte Abstand und die Mindestpressung nicht eingehalten werden.

Während bei bekannten Verschlüssen häufig breite und eher flache Innenrippen eingesetzt werden, so ist erfindungsgemäß eine andere, schmalere Ausbildung der Innenrippen vorgesehen, welche zu einem deutlich kleineren Verdrängungsvolumen an dem Dichtelement führt, insbesondere bei einer ansonsten gleichen Anzahl an Innenrippen. Hierdurch kann die Neigung eines in der Verschlusskappe montierten Dichtelements zu einem axialen Versatz stark reduziert werden, so dass das geforderte Abstandmaß nach Setzen des Dichtelements präziser eingehalten werden kann. Ein großes Verdrängungsvolumen führt dagegen zu einer starken Kompression des Dichtelements und begünstigt damit den unerwünschten axialen Versatz.

Der axiale Versatz des Dichtelements in der Öffnung der Verschlusskappe kann durch die erfindungsgemäß gestaltete Innenrippe auf weniger als 0,3 mm, bevorzugt auf weniger als 0,2 mm und besonders bevorzugt auf weniger als 0,15 mm begrenzt werden.

Die Innenrippe weist hierzu eine Breite von weniger als 1 mm, bevorzugt von weniger als 0,9 mm und besonders bevorzugt von weniger als 0,8 mm auf. Besonders günstig sind weiterhin die Breite oder der Querschnitt der Innenrippe über einen Großteil der Länge der Innenrippe konstant, insbesondere über eine Länge von wenigstens 50 % der Länge der Innenrippe, bevorzugt über eine Länge von wenigstens 60 % und besonders bevorzugt von wenigstens 65 %. Auch dieses reduziert deutlich die Neigung zu einem axialen Versatz des Dichtelements in der Verschlusskappe.

Auf diese Weise ist es möglich, die erforderliche Mindestpressung zum Verschließen der Öffnung einzuhalten. So kann eine Verschlusskappe zur Verfügung gestellt werden, bei der der axiale Abstand zwischen dem äußeren Anschlag und dem inneren Anschlag der Verschlusskappe, 2,85 mm mit einer Toleranz von +/- 0,2 mm, bevorzugt von +/- 0,1 mm und besonders bevorzugt von +/- 0,05 mm beträgt. Damit kann die geforderte Mindestpressung zur Abdichtung der Öffnung sicher eingehalten werden.

Der Betätigungsabschnitt kann weiterhin besonders günstig zumindest zwei längliche, in ihrer Hauptausrichtung axial angeordnete, radial nach Außen von der Mantelfläche hervorstehende Außenrippen umfassen, welche für einen Benutzer eine bessere Greifmöglichkeit bieten und so das Auf- bzw. Abschrauben erleichtern. Von Vorteil sind hier schmale Außenrippen vorgesehen, welche sich über nahezu die gesamte Länge des Betätigungsabschnitts erstrecken. In einer günstigen Ausführungsform weisen diese Außenrippen eine Länge, also eine Ausdehnung in axialer Richtung, von wenigstens 4,5 mm, bevorzugt von wenigstens 5,0 mm auf. Die Höhe dieser Außenrippen, also der radiale Überstand gegenüber der Mantelfläche, liegt günstigerweise bei wenigstens 0,3 mm, so dass der Benutzer eine stabile Griffffläche vorfindet und somit den Verschluss besser und leichter öffnen kann.

Die Zuverlässigkeit und die Einsatzmöglichkeit des mit dem erfindungsgemäßen Verschluss versehenen Behälters für pharmazeutische Präparate wird weiterhin durch die Farbstabilität des Behälters und insbesondere des mit dem erfindungsgemäßen Verschluss versehenen Behälters bestimmt. Da Verschlüsse typischerweise in hohen Stückzahlen mittels Spritzgussverfahren hergestellt werden, kommen als Materialien hierfür im Wesentlichen geeignete thermoplastische Kunststoffe in Frage.

Die Farbstabilität ist insofern von großer Bedeutung, als dass der Verschluss und/oder der Behälter mit dem Verschluss einer Behandlung zur Sterilisation unterzogen werden können, welche zu Farbveränderungen oder sonstigen Materialveränderungen der beteiligten Kunststoffe führen können. Die Sterilisation dient dazu, pharmazeutische Verpackungen, also beispielsweise Behälter mit pharmazeutischen Präparaten, von möglichst allen enthaltenen oder anhaftenden Mikroorganismen zu befreien.

Verschiedene Behandlungsmethoden zur Sterilisation stehen zur Verfügung, etwa die Sterilisation unter Heißluft. Hierbei handelt es sich um ein physikalisches Sterilisationsverfahren, bei dem thermostabile Medizinprodukte trockener Hitze ausgesetzt werden. Zur Befreiung der Produkte von Keimen wie Bakterien, Pilzen, Viren oder Sporen sind Temperaturen von mindestens 180 °C über einen Zeitraum von mindestens 30 Minuten notwendig.

Ferner ist das Autoklavieren als am häufigsten eingesetztes Sterilisationsverfahren bekannt. Hierbei wird Wasserdampf mit Temperaturen von beispielsweise 120 °C bei 0,5 bar genutzt. Empfindliche Produkte können somit länger im Autoklav verweilen als bei der Heißluft-Sterilisation.

Zusätzlich verteilt sich der Wasserdampf besser auf der Produktoberfläche, was zum besseren Erreichen der erforderlichen Sterilisationstemperatur über das ganze Produkt beiträgt.

Weiterhin bekannt ist auch die Bestrahlung mit elektromagnetischer Strahlung, beispielsweise Gamma-Strahlen oder Röntgen-Strahlen. Bei der sog. Gammasterilisation werden die pharmazeutischen Präparate etwa mittels Gamma-Strahlen einer bestimmten Energie-Dosis bestrahlt. Bei Verwendung von Gamma-Strahlen oder Röntgen-Strahlen erfolgt die Bestrahlung im Allgemeinen mit einer Energie-Dosis im Bereich von 25-40 kGy.

Die meisten bekannten Methoden zur Sterilisation schränken die Auswahl der Materialien, etwa des Materials für den Verschluss, dahingehend ein, dass bestimmte Auswirkungen, etwa auf die Farbe oder die Konsistenz des Materials, minimiert werden. In anderen Worten, eine Bestrahlung des Verschlusses kann beispielsweise die Polymerstruktur eines verwendeten Kunststoffes verändern, was dann in der Folge zu Farbveränderungen führen kann. Auch können bestimmte empfindliche thermoplastische Kunststoffe unter den teilweise geforderten hohen Temperaturen leiden und beispielsweise brüchig werden oder ebenfalls die Farbe ändern.

Während für bestimmte Anwendungsbereiche eine Farbveränderung infolge einer Sterilisation durchaus gewünscht sein kann, so wird im Rahmen dieser Erfindung davon ausgegangen, dass eine Farbveränderung des Verschlusses unerwünscht ist. Unabhängig von der Art der Sterilisation, d.h. also bei Anwendung zumindest einer der vorstehend genannten Methoden zur Sterilisation, soll möglichst kein "Farb-Shift", also keine Farbveränderung des Verschlusses erfolgen. Im Sinne der vorliegenden Erfindung wird eine Farbveränderung dann als unerwünscht bezeichnet, wenn der Farbort des Materials nach der Sterilisation von demjenigen vor der Sterilisation um einen Wert ΔE, bestimmt als Farbortänderung im CIE 1931-System, um mehr als ΔE = 0,05 abweicht. Die Farbortänderung ΔE ist dabei gegeben durch die folgende Formel:

$$\Delta E = \sqrt{(x^2 + y^2)}$$

Erfindungsgemäß wird daher in einer bevorzugten Ausführungsform für den Verschluss ein Material ausgewählt, welches sich infolge der Sterilisation kaum oder vorzugsweise nicht farblich ändert.

Dies erfolgt höchst überraschend durch die Auswahl von bestimmten Farbpigmenten und Antioxidationsmittel für das Material zur Herstellung des Verschlusses. Die Erfinder haben herausgefunden, dass eine ganz bestimmte Materialzusammensetzung, umfassend bestimmte ausgewählte Pigmente, welche eine grau-grüne Farbanmutung des Verschlusses bewirken, nicht oder zumindest kaum dazu tendiert, bei Anwendung einer der oben genannten Sterilisationsmethoden die Farbe zu ändern. Stattdessen wird die ursprüngliche Farbe zumindest nahezu beibehalten und es kommt vorzugsweise nicht zu einem Farb-Shift. Diese Farbe entspricht der Farbe RAL 7009 nach dem RAL-Farbsystem.

Diese überraschende farbstabile Wirkung wird dadurch erreicht, dass eine Zusammensetzung aus farbgebenden Pigmenten gefunden werden konnte, welche zu einer Polymerstruktur des Kunststoffes führen, die stabil ist sowohl bei erhöhter Temperatur als auch bei einer Beaufschlagung mit elektromagnetischer Strahlung wie vorstehend angegeben. Ein Material zur Herstellung des Verschlusses umfasst die folgenden Pigmente als farbgebende Elemente:

- Titandioxid Kronos® 2233
- Ruß PRINTEX® F 85
- 6975 Titanorange
- VYNAMON ® Green 600734

Ein Verschluss aus einem Material, welches zur Farbgebung diese Pigmente umfasst, ist daher auch für Verfahren zur Sterilisation geeignet, welche mit Gamma- oder Röntgenstrahlen arbeiten. Es hat sich gezeigt, dass derartige Verschlüsse bei einer Bestrahlung mit einer Energie-Dosis in einem Bereich von 25-40 kGy zu keinen oder zu kaum mit dem menschlichen Auge wahrnehmbaren Farbveränderungen führen und damit als farbstabil gelten können.

Besonders vorteilhaft ist es, dass eine derartige Materialzusammensetzung auch bei anderen Sterilisationsmethoden, etwa einer Heißdampf-Sterilisation bei bis zu 134 °C für eine Zeitdauer von 10 Minuten, zu keinen oder zu kaum mit dem menschlichen Auge wahrnehmbaren Farbveränderungen führt.

Dies ist besonders günstig, da die Auswahl der anzuwendenden Sterilisationsmethode damit weitgehend unabhängig von dem Material des Verschlusses ausgewählt werden kann. Auch eine Kombination der oben genannten Sterilisationsmethoden führt nicht zu einem Nachlassen der Dichtigkeit. Insbesondere weist der Verschluss nach der Sterilisation eine Dichtigkeit bis 4 bar für 30 Sekunden auf und verfügt über ein Öffnungsdrehmoment weniger als 15 N*cm.

Gegenstand der Erfindung ist schließlich in einem weiteren Aspekt ein Verfahren zum kontaminationsfreien lösbaren Verschließen eines distalen Endes eines zylinderförmigen Behälters für pharmazeutische Präparate, vorzugsweise eine Spritze, insbesondere mit einem Verschluss wie vorstehend beschrieben.

Die Erfindung wird nachfolgend anhand bevorzugter Ausführungsformen und unter Bezugnahme auf die beigefügten Figuren detaillierter beschrieben.

Die Zeichnungen zeigen:

Fig. 1 einen Längsschnitt durch einen Verschluss nach dem Stand der Technik,

Fig. 2 einen Längsschnitt durch einen erfindungsgemäßen Verschluss,

Fig. 3 einen Längsschnitt durch einen Behälter für pharmazeutische Präparate, welcher über eine Luer-Lock-Befestigung verfügt, mit einem teilweise montierten Verschluss,

Fig. 4 einen Verlauf des Drehmoments beim Öffnen eines erfindungsgemäßen Verschlusses zum Lösen von dem Behälter,

Fig. 5 einen Verlauf des Drehmoments beim Öffnen eines aus dem Stand der Technik bekannten Verschlusses zum Lösen von dem Behälter,

Fig. 6 in einer Draufsicht einen Ausschnitt eines erfindungsgemäßen Verschlusses mit einer Aufwölbung,

Fig. 7 einen Längsschnitt durch einen erfindungsgemäßen Verschluss mit einem Dichtelement,

Fig. 8 einen Längsschnitt durch einen erfindungsgemäßen Verschluss ohne Dichtelement,

Fig. 9 in einer Schrägansicht einen erfindungsgemäßen Verschluss.

Detaillierte Beschreibung bevorzugter Ausführungsformen

Bei der nachfolgenden detaillierten Beschreibung bevorzugter Ausführungsformen bezeichnen um der Klarheit willen gleiche Bezugszeichen im Wesentlichen gleiche Teile in oder an diesen Ausführungsformen. Zur besseren Verdeutlichung der Erfindung sind die in den Figuren dargestellten bevorzugten Ausführungsformen jedoch nicht immer maßstabsgerecht gezeichnet.

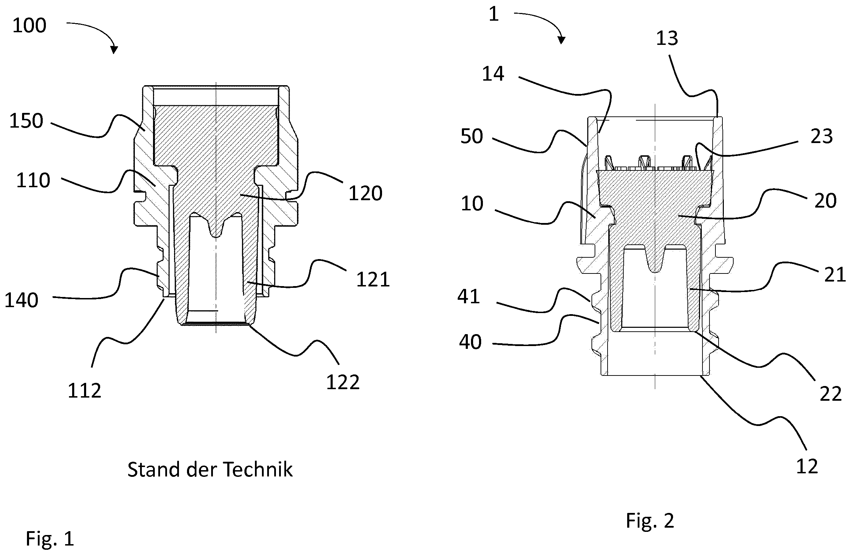

Fig. 1 zeigt einen Längsschnitt durch einen Verschluss 100 nach dem Stand der Technik. Dieser Verschluss 100 umfasst eine zylindrische Verschlusskappe 110 mit einem zylindrischen Hohlraum zur Aufnahme eines Dichtelements 120, wobei die Verschlusskappe 110 einen Montageabschnitt 140, welcher in montierter Position zu dem Behälter hinweist, und einen Betätigungsabschnitt 150 umfasst.

In den zylindrischen Hohlraum der Verschlusskappe 110 ist das Dichtelement 120 eingefügt, welches über einen Dichtabschnitt 121 verfügt. Deutlich erkennbar ist, dass die Außenkante 122 des Dichtabschnitts über der Außenkante 112 der Verschlusskappe 110 hervorsteht. Dies ist für eine automatisierte Montage ungünstig, da es zu einem Verkippen und Verkanten während des Aufsetzens zu Beginn der Montage kommen kann, wenn das Dichtelement zuerst in Kontakt mit dem Behälter, insbesondere mit der Spitze des Behälters, gelangt. So ist auch eine Zentrierung des Verschlusses beim Einschraubvorgang kaum möglich, da das Dichtelement 120 aus einem nachgiebigen elastomeren Material gefertigt ist. Gerade bei vollautomatisierten Montageprozessen und den geforderten hohen Stückzahlen kann hier wertvolle Zeit verloren gehen, wenn die Montage vor diesem Hintergrund besonders langsam erfolgen muss oder, etwa nach einem Verkanten, wiederholt werden muss.

Fig. 2 zeigt einen Längsschnitt durch einen erfindungsgemäße Verschluss 1 zum lösbaren Verschließen einer Öffnung am distalen Ende eines zylinderförmigen Behälters für pharmazeutische Präparate (nicht dargestellt), insbesondere eine Spritze, vorzugsweise mittels einer Luer-Lock-Befestigung. Der erfindungsgemäße Verschluss 1 umfasst eine zylindrische Verschlusskappe 10 mit einem durchgehenden zylindrischen Hohlraum 14 zur Aufnahme eines Dichtelements 20, wobei die Verschlusskappe 10 einen Montageabschnitt 40, welcher in montierter Position zu dem Behälter hinweist, und einen Betätigungsabschnitt 50 umfasst.

Der Montageabschnitt 40 umfasst ein Außengewinde 41, welches in montierter Position in das Außengewinde der Luer-Lock-Befestigung des Behälters eingreift und eine lösbare Verbindung mit diesem schafft. Der Betätigungsabschnitt 50 ist mit Außenrippen zur Handhabung durch einen Benutzer ausgestattet. Der zylindrische Hohlraum 14 der Verschlusskappe 10 ist durchgehend ausgebildet zur Aufnahme des in dem Beispiel einstückigen Dichtelements 20.

Gut zu erkennen ist in der Darstellung der deutliche Rückversatz der Außenkante 22 des Dichtelements 20 gegenüber der Außenkante 12 des Montageabschnittes 40 der Verschlusskappe 10.

Der erfindungsgemäße Rückversatz ermöglicht es in verblüffend einfacher Weise, den Montageprozess deutlich zu verbessern und die Prozesssicherheit zu erhöhen, da ein Verkippen und Verkanten während des Aufsetzens des Verschlusses 1 auf den Behälter oder zu Beginn des Montageprozesses wirksam verhindert werden kann. Dies ist dem Umstand geschuldet, dass während des Fügens zunächst eine Zentrierung des Verschlusses 1 über den Montageabschnitt 40 erfolgt, bevor das Dichtelement 20 in Kontakt mit dem Behälter, insbesondere mit der Spitze des Behälters, gelangt. Gerade bei vollautomatisierten Montageprozessen und den geforderten hohen Stückzahlen ist eine Erhöhung der Montagesicherheit von großer Bedeutung für die Effizienz der Herstellung und kann helfen, wertvolle Zeit in der Montage einzusparen.

Der Rückversatz des Dichtelements 20 gegenüber der Außenkante 12 des Montageabschnittes 40 der Verschlusskappe 10 erhöht also die Prozesssicherheit, da eine Zentrierung des Verschlusses 1 beim Einschraubvorgang bereits vor einem Kontakt des Dichtelements 20 mit dem Behälter erfolgt. Vorliegend erfolgt der erste Kontakt des Dichtelements 20 mit der Spitze des Behälters also erst nach einer Zentrierung von Verschluss 10 und Behälter, so dass ein rein axiales Aufschieben möglich ist, was weniger fehleranfällig ist.

Gleichzeitig führt der Rückversatz auch zur einer Verringerung der Kontamination anderer Verschlüsse oder auch des Behälters durch Fertigungsrückstände, welche auf oder an dem Dichtelement 20 vorhanden sein können. Dies können beispielsweise in der Fertigung der Dichtelemente verwendete Flüssigkeiten, etwa Silikonöl, sein. Gerade bei Dichtelementen, welche wie vorliegend aus einem elastomeren Material gefertigt sind, ist dies häufig der Fall.

Der Rückversatz beträgt in dem Beispiel etwa 2,5 mm. Die Gefahr einer Kontaminierung kann bereits reduziert werden, wenn der Rückversatz wenigstens 0,5 mm, bevorzugt wenigstens 1,0 mm und besonders bevorzugt wenigstens 1,5 mm beträgt. In Verbindung mit den gegebenen Toleranzen ergibt sich ein günstiger Rückversatz weiterhin in einem Bereich von 1,5 bis 2,0 mm, von 2,0 bis 2,5 mm oder von 2,5 bis 3,0 mm.

In der Fig. 2 ist ferner zu sehen, dass auch ein Rückversatz der Außenkante 23 des Dichtelements 20 gegenüber der Außenkante 13 der Verschlusskappe 10 vorgesehen ist. Dieser Rückversatz beträgt vorzugsweise ebenfalls wenigstens 0,5 mm, bevorzugt wenigstens 1 mm und besonders bevorzugt wenigstens 1,5 mm beträgt. Im Beispiel beträgt der Rückversatz etwa 2,5 mm und ist damit in etwa so groß wie der Rückversatz auf der gegenüberliegenden Seite.

Der beidseitige Rückversatz der Außenkanten 22, 23 des Dichtelements 20 gegenüber den Außenkanten 12, 13 der Verschlusskappe 10 verhindert demnach zuverlässig eine gegenseitige Kontaminierung nebeneinanderliegender Verschlüsse, etwa in einer Transportverpackung. Konstruktiv gelöst wird dies durch eine reduzierte Länge des Dichtelements 20 gegenüber der Länge der Verschlusskappe 10. Im Beispiel beträgt das Verhältnis der Länge der Dichtelements zur Länge der Verschlusskappe etwa 70 %. Als vorteilhaft hat sich ein Verhältnis der Länge des Dichtelements 20 zur Länge der Verschlusskappe 10 von höchstens 95 %, bevorzugt höchstens 90 % und besonders bevorzugt höchstens 85 % herausgestellt.

Das Montage- und Demontageverhalten des Verschlusses 1 auf dem Behälter wird zu einem wesentlichen Anteil durch die Ausbildung des Außengewindes der Verschlusskappe 10 bestimmt.

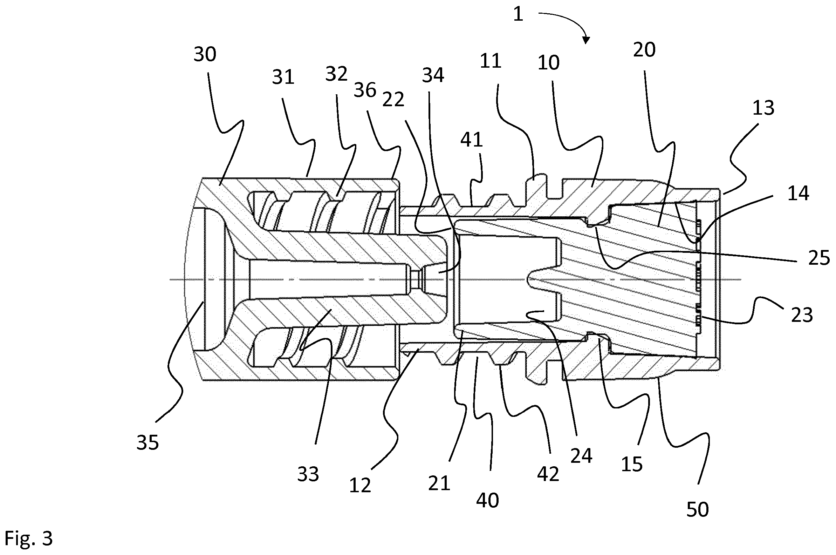

Fig. 3 zeigt in einem Längsschnitt einen Behälter 30 für pharmazeutische Präparate, welcher über eine Luer-Lock-Befestigung verfügt, mit einem teilweise montierten Verschluss 1. Der Behälter 30 umfasst an seinem distalen Ende eine Manschette 31, welche mit einem Innengewinde 32 ausgebildet ist und die Luer-Lock-Befestigung darstellt. Die Manschette 31 umschließt eine konisch zulaufende Spitze 33 am distalen Ende des Behälters, welche eine Öffnung 34 aufweist, etwa zum Austreiben eines Präparates aus der Kammer 35.

Das Außengewinde 41 des Montageabschnitts 40 ist passgenau gegengleich zu dem Innengewinde 32 ausgebildet. Das Außengewinde 41 ist gemäß der gängigen Norm ISO 594-2 ausgebildet, wobei erfindungsgemäß aber eine andere Form der Rippe 42 vorgesehen ist. Die erfindungsgemäße Rippe 42 ist schmaler und/oder mit einer steileren Flanke ausgebildet, was zu einem schmaleren Gewindegang und damit zu einem größeren Abstand der benachbarten Gewindeflanken zueinander führt. Dies führt zunächst zu einem einfacheren Aufschrauben des Verschlusses 1 auf den Behälter 30, da die wirkenden Reibungskräfte geringer sind.

Dies ermöglicht es ferner, auf der Gewindeflanke an bestimmten Positionen Erhebungen, vorzugsweise in Punktform (nicht dargestellt), vorzusehen, vorzugsweise jeweils angeordnet an zwei gegenüberliegenden Positionen der Gewindeflanke. Zwei gegenüberliegend angeordnete Erhebungen können damit ein Erhebungspaar ausbilden. Ein derartiges Erhebungspaar ist dabei derart ausgebildet, dass in montierter Position die Erhebungspunkte dann an der Gewindeflanke der Luer-Lock-Befestigung anliegen und leicht gestaucht bzw. deformiert werden. Damit geht eine zusätzliche Kraftkomponente in die Verbindung ein.

Diese Kraftkomponente wirkt gleichermaßen auch bei dem Öffnen des Verschlusses und ermöglicht es damit, einen vorgegebenen Schwellwert für das Öffnen des Verschlusses präzise bereit zu stellen. Auf diese Weise kann eine bessere, für den Benutzer einfachere Charakteristik für das Öffnungsdrehmoment bereitgestellt werden. Das Außengewinde 41 umfasst in dem Beispiel insgesamt vier Erhebungspaare (nicht dargestellt).

Auf diese Weise wird weiter begünstigt, das für die Montage und/oder das Lösen erforderliche Drehmoment präzise einstellen zu können, sowie auch einen vorbestimmten Kräfteverlauf während der Drehbewegung sicherzustellen.

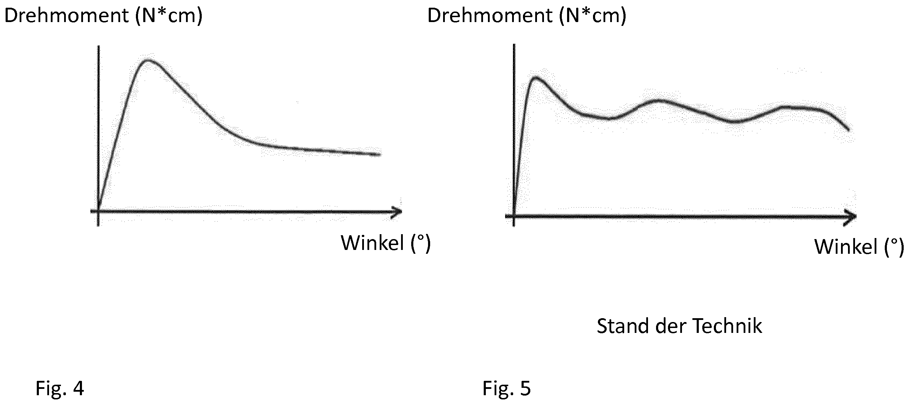

Die Fig. 4 und 5 zeigen anhand von Ausführungsbeispielen den Verlauf des Drehmoments beim Öffnen des Verschlusses 1 zum Lösen von dem Behälter 30. In Fig. 5 ist eine Charakteristik für das Öffnungsdrehmoment eines Verschlusses 100 nach dem Stand der Technik dargestellt, in Fig. 4 die Charakteristik für das Öffnungsdrehmoment für einen erfindungsgemäßen Verschluss 1.

Es ist deutlich zu erkennen, dass beim Öffnen des erfindungsgemäßen Verschlusses 1 zu Beginn eine höhere Kraftkomponente aufgewendet werden muss, um das Lösen zu bewirken. Wird ein Schwellwert überschritten, sinkt die einzubringende Kraftkomponente kontinuierlich ab. Dies erleichtert das Öffnen des Behälters 30 und das Lösen des Verschlusses 1 für den Benutzer. Das aufzubringende Drehmoment richtet sich demnach nach dem Öffnungswinkel. Nach Überwinden eines Anfangswiderstandes bei dem Lösen, welches sich infolge der Erhebungen einstellt, sinkt die einwirkende Kraftkomponente aufgrund der schmaleren Gewindegänge, welche mehr Freiraum bieten, so dass die Erhebungen weniger gestaucht werden. Auf diese Weise ist es auch möglich, ein präzises Drehmoment für das Öffnen, beispielsweise von 16 N*cm mit einer Genauigkeit von +/-0,5 N*cm, einzustellen.

Damit unterscheidet sich das Löseverhalten des erfindungsgemäßen Verschlusses 1 von aus dem Stand der Technik bekannten Verschlüssen 100, bei welchen die zu Beginn des Lösens aufzuwendende Kraftkomponenten während des Lösens des Verschlusses kaum sinkt und bis zum Ende hin auf einem hohen Niveau verbleibt. Anhand des Kraftverlaufs in Fig. 5 ist dies gut zu erkennen. Dies ist dem Umstand geschuldet, dass der Verschluss 100 eine Aufwölbung des Gewindegangs für eine zusätzliche Kraftkomponente vorsieht, welche dann aber während des gesamten Lösens einwirkt. Für den Benutzer ist diese Charakteristik des zum Lösen aufzubringenden Drehmoments eher ungünstig, da für nahezu das gesamte Öffnen ein höheres Moment aufgebracht werden muss.

Erfindungsgemäß wird die Montage des Verschlusses weiterhin dadurch vereinfacht, dass der Gewindegang 41 zum einen schmaler ausgeführt ist, was die mögliche Winkelorientierung, also die winkelrichtige Ausrichtung von Verschluss und Behälter zueinander zu Beginn der Montage, vergrößert und damit erleichtert. Um diesen Toleranzbereich der geforderten Winkelorientierung noch stärker zu erweitern, ist in einer bevorzugten Ausführungsform eine Aufwölbung des neben dem Gewindeeingang liegenden Abschnittes des Gewindegangs zur Vergrößerung des Freiraumes von der Außenkante bis zu dem ersten Gewindegang vorgesehen, wodurch sich der Toleranzbereich der Winkelorientierung nochmals vergrößern lässt.

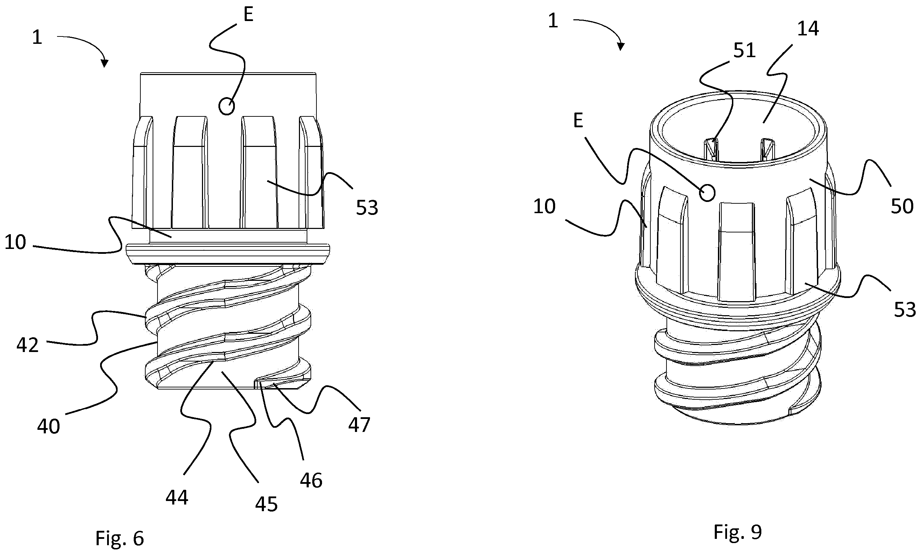

Fig. 6 zeigt in einer Draufsicht einen Ausschnitt eines Verschlusses 1 mit einer Aufwölbung 44. Dieser leichte axialer Versatz des Gewindegangs, welcher die Aufwölbung 44 darstellt, bildet einen zusätzlichen Freiraum 45 in einem Abschnitt aus, welcher benachbart zu dem Gewindeeingang angeordnet ist. Hierdurch wird der Toleranzbereich der Winkelorientierung vergrößert. In anderen Worten wird derjenige Bereich, der die erforderliche Orientierung von Verschluss 1 und Behälter 30 vor dem Fügen zueinander angibt, erweitert. Die erfindungsgemäße Gestaltung des Gewindeeinstiegs in dem abgebildeten Ausführungsbeispiel ermöglicht es, diese Winkeltoleranz auf einen Bereich von +/- 10° zu erweitern. Erfindungsgemäß ist es möglich, die Winkeltoleranz in einem Bereich zwischen +/- 5° und +/-15°, bevorzugt zwischen +/- 8° und +/- 12° festzulegen. Dies führt zu einem deutlich günstigeren Montageverhalten.

In einer ebenfalls günstigen Ausführungsform ist der Gewindeeingang 46 zusätzlich mit einer Fase 47 ausgebildet, um das Fügen nochmals zu erleichtern.

Die Zuverlässigkeit des mit dem erfindungsgemäßen Verschluss 1 versehenen Behälters 30 wird weiterhin durch die Passgenauigkeit derjenigen Komponenten, welche die Dichtheit der Öffnung 34 des Behälters 30 bewirken, beeinflusst. Von entscheidender Bedeutung ist hier, wie aus der Fig. 3 ersichtlich, der axiale Abstand zwischen einem radial nach außen hervorstehenden Anschlag 11 der Verschlusskappe 10, welcher in montierter Position an dem Behälter 30 anliegt, insbesondere an dem Kragen 36 der Luer-Lock-Befestigung 32, und der Innenkante 24 eines Dichtabschnitts 21 des Dichtelements 20, welcher die Abdichtung der Öffnung 34 der Spitze 33 bewirkt. Der Dichtabschnitt 21 liegt in montierter Position eng an der Spitze 33 an und umschließt die Öffnung 34, wobei die Innenkante 24 mit einer vorgegebenen Mindestpressung an der Öffnung 34 anliegt.

Zur axialen Positionierung des Dichtelements 20 in der Öffnung 14 der Verschlusskappe 10 ist diese mit einer radial nach innen vorspringenden, umlaufenden Schulter 15 ausgebildet. Das Dichtelement 20 weist auf seiner Mantelfläche eine ebenfalls umlaufende Ausnehmung 25 auf, welche derart positioniert ist, dass in montierter Position die Schulter 15 in die Ausnehmung 25 eingreift und einen axialen Anschlag bewirkt. Auf diese Weise kann eine präzise Positionierung des Dichtelements 20 und damit der Innenkante 24, welche die Öffnung 34 des Behälters 30 (nicht dargestellt) abdichtet, erreicht werden.

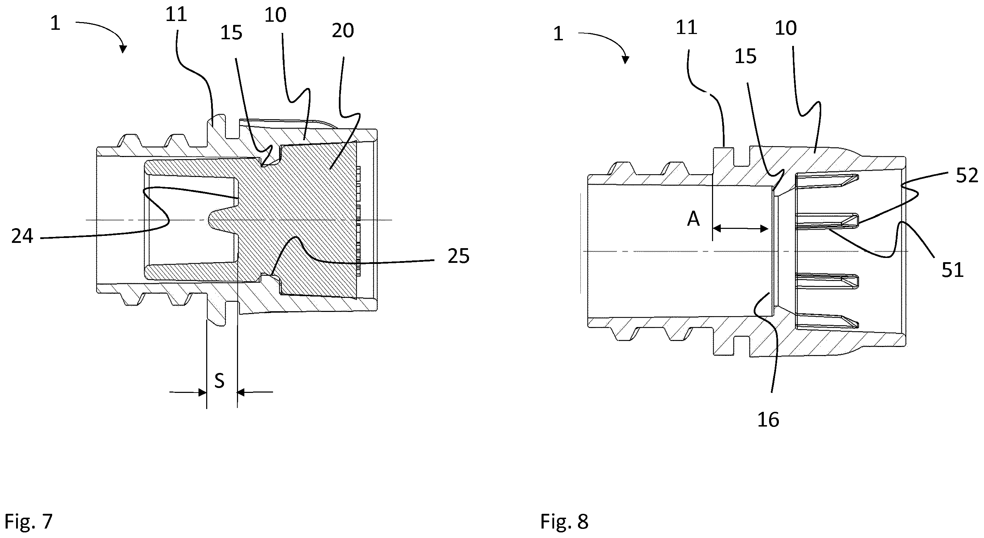

Fig. 7 zeigt diese Zusammenhänge anhand eines Längsschnittes durch einen erfindungsgemäßen Verschluss 1 mit einem Dichtelement 20. In der Darstellung ist mit "S" das sogenannte Setzmaß angegeben, also der Abstand, der sich in montierter Position des Verschlusses zwischen der Innenkante 24 des Dichtelements 20 und dem Behälter 30 einstellt. Dieser ist entscheidend, um die geforderte Mindestpressung zur Abdichtung der Öffnung zu erreichen.

Fig. 8 verdeutlicht diese Zusammenhänge nochmals anhand eines Längsschnittes durch einen erfindungsgemäßen Verschluss 1, welcher in der Darstellung kein Dichtelement 20 aufweist. Um dieses Setzmaß einzuhalten, ist ein bestimmter Abstand "A" zwischen der zum Behälter hinweisenden Außenkante des nach außen hervorstehenden Anschlags 11 der Verschlusskappe 10, welche also in montierter Position auf dem Kragen des Behälters aufliegt, und einem durch die Schulter 15 definierten Anschlag 16 innerhalb der Öffnung 14 der Verschlusskappe 10 gefordert. Im Beispiel ist hier ein Montagemaß von A = 2,85 mm mit einer sehr geringen Toleranz gefordert. Der innere Anschlag 16 bewirkt dabei die zur Abdichtung aufzubringende Mindestpressung des Dichtelements 20 auf die Öffnung.

Die Verschlusskappe 10 ist derart ausgelegt, dass eine dünne und vor allen Dingen gleichmäßige Materialdicke der Seitenwandung über möglichst die gesamte Länge der Verschlusskappe gegeben ist, um eine hohe Maßgenauigkeit zu erreichen. Daher liegt die Wandstärke der Verschlusskappe 10, also die Dicke der Wandung, an der dicksten Stelle bei höchstens 2,0 mm. Dabei schwankt die Dicke der Wandung auf die gesamte Länge der Verschlusskappe gesehen um nicht mehr als 0,5 mm, bevorzugt um nicht mehr als 0,3 mm.

Ferner wird erfindungsgemäß das Spritzgussverfahren dahingehend optimiert, dass ein Einspritzpunkt gewählt wird, welcher im Bereich des Betätigungsabschnitts 50 liegt. Die Fig. 9 zeigt in einer Schrägansicht einen erfindungsgemäßen Verschluss 1, wobei der Einspritzpunkt in für das Spritzgussverfahren dem abgebildeten Beispiel mit dem Buchstaben "E" gekennzeichnet ist. Der Einspritzpunkt liegt damit außenseitig auf der Mantelfläche des Betätigungsabschnitts 50 axial beabstandet von einer Außenrippe 53. Hierdurch kann zudem einer möglichen Verletzungsgefahr für einen Benutzer entgegengewirkt werden, da durch den Anguss scharfe Kanten entstehen können, welche bei einem Zugreifen hervorstehen. Vorliegend liegen diese potentiellen Angusskanten außerhalb des Griffbereiches.

Die drehsichere Halterung des Dichtelements 20 in der durchgehenden Öffnung 14 der Verschlusskappe wird über längliche, in ihrer Hauptausrichtung axial angeordnete und radial nach Innen hervorstehende Innenrippen 51 auf der Innenwandung der Öffnung 14 des Betätigungsabschnittes 50 erreicht. Diese sind für eine gleichmäßige Pressung in gleichmäßigen Abständen über den Innenumfang verteilt angeordnet. Im abgebildeten Beispiel sind insgesamt 8 derartige Innenrippen 51 vorgesehen.

Erfindungsgemäß ist eine schmale Ausbildung der Innenrippen 51 vorgesehen, welche zu einem deutlich kleineren Verdrängungsvolumen an einem eingesetzten Dichtelement 20 führen. Hierdurch wird die Neigung eines in der Verschlusskappe 10 montierten Dichtelements 20 zu einem axialen Versatz stark reduziert, so dass das geforderte Abstandmaß nach Setzen des Dichtelements, also das Setzmaß zur Einhaltung der Mindestpressung, präzise eingehalten werden kann. Der axiale Versatz des Dichtelements in der Öffnung der Verschlusskappe wird durch die erfindungsgemäß gestaltete Innenrippe auf weniger als 0,3 mm, bevorzugt auf weniger als 0,2 mm und besonders bevorzugt auf weniger als 0,15 mm begrenzt.

Die Innenrippe 51 weist daher in dem abgebildeten Beispiel eine Breite von etwa 0,9 mm auf. Ohne Beschränkung auf das Ausführungsbeispiel sind Breiten der Innenrippe 51 von weniger als 1 mm, bevorzugt von weniger als 0,9 mm und besonders bevorzugt von weniger als 0,8 mm günstig. Für einen stabilen Sitz sind weiterhin die Breite und die Querschnittsform der Innenrippe 51 über einen Großteil der Länge der Innenrippe 51 konstant, wie beispielsweise aus der Fig. 8 gut zu erkennen. In andere Worten, bis auf eine kleine Einführungsschräge 52 zum Setzen des Dichtelements ist der Querschnitt der Rippe über die Länge konstant. Es hat sich als günstig herausgestellt, wenn die Querschnittsform der Innenrippe 51 über eine Länge von wenigstens 50 % der gesamten Länge der Innenrippe 51, bevorzugt über eine Länge von wenigstens 60 % und besonders bevorzugt von wenigstens 65 % gleich ist. Dieses reduziert deutlich die Neigung des Dichtelements 20 zu einem späteren axialen Versatz.

Auf diese Weise ist es möglich, die erforderliche Mindestpressung zum Verschließen der Öffnung 34 einzuhalten. Damit wird eine Verschlusskappe 10 zur Verfügung gestellt, bei der der axiale Abstand zwischen dem äußeren Anschlag 11 und dem inneren Anschlag der Verschlusskappe 2,85 mm mit einer Toleranz von +/- 0,2 mm, bevorzugt von +/- 0,1 mm und besonders bevorzugt von +/-0,05 mm beträgt. Auf diese Weise wird die geforderte Mindestpressung zur Abdichtung der Öffnung 34 sicher eingehalten.

Der Betätigungsabschnitt 50 umfasst weiterhin besonders günstig längliche, in ihrer Hauptausrichtung axial angeordnete, radial nach Außen von der Mantelfläche hervorstehende Außenrippen 53, welche für einen Benutzer eine bessere Greifmöglichkeit bieten und so das Auf- bzw. Abschrauben erleichtern. Im Beispiel umfasst der Betätigungsabschnitt 50 acht derartige Außenrippen 53. Diese Außenrippen 53 erstrecken sich über nahezu die gesamte Länge des Betätigungsabschnitts 50. In einer günstigen Ausführungsform weisen diese Außenrippen eine Länge, also eine Ausdehnung in axialer Richtung, von wenigstens 4,5 mm, bevorzugt von wenigstens 5,0 mm auf. Im Beispiel beträgt die Länge etwa 5,3 mm, was bereits zu einer hinreichenden Grifffläche führt.

Die Höhe dieser Außenrippen 53, also der radiale Überstand gegenüber der Mantelfläche, liegt bei wenigstens 0,3 mm, so dass der Benutzer eine stabile Grifffläche vorfindet und somit den Verschluss besser und leichter öffnen kann.

Der Verschluss 1 ist aus einem farbstabilen Kunststoff gefertigt. Zur Farbgebung des Kunststoffes kommen die nachfolgend genannten Pigmente als farbgebende Elemente zum Einsatz:

- Titandioxid Kronos® 2233
- Ruß PRINTEX® F 85
- 6975 Titanorange
- VYNAMON ® Green 600734

Ein Verschluss 1 aus einem derartigen Material ist daher auch für Verfahren zur Sterilisation geeignet, welche mit Gamma- oder Röntgenstrahlen arbeiten. Es hat sich gezeigt, dass derartige Verschlüsse bei einer Bestrahlung mit einer Energie-Dosis in einem Bereich von 25-40 kGy zu keinen oder zu kaum mit dem menschlichen Auge wahrnehmbaren Farbveränderungen führen und damit als farbstabil gelten können.

Eine derartige Materialzusammensetzung ist auch für andere Sterilisationsmethoden geeignet, etwa einer Heißdampf-Sterilisation bei bis zu 134 °C für eine Zeitdauer von 10 Minuten, welche zu keinen oder zu kaum mit dem menschlichen Auge wahrnehmbaren Farbveränderungen führt.

Bezugszeichenliste:

| 1 | Verschluss |
|---|---|
| 10 | Verschlusskappe |
| 11 | Anschlag |
| 12 | Außenkante der Verschlusskappe |
| 13 | Außenkante der Verschlusskappe |
| 14 | Hohlraum |
| 15 | Schulter |
| 16 | Anschlag |
| 20 | Dichtelement |
| 21 | Dichtabschnitt |
| 22 | Außenkante des Dichtabschnittes |
| 23 | Außenkante des Dichtabschnittes |
| 24 | Innenkante |
| 25 | Ausnehmung |
| 30 | Behälter |
| 31 | Manschette |
| 33 | Spitze |
| 34 | Öffnung |
| 35 | Kammer |
| 36 | Kragen |
| 40 | Montageabschnitt |
| 41 | Außengewinde |
| 42 | Rippe |
| 44 | Aufwölbung |
| 45 | Freiraum |
| 46 | Gewindeeingang |
| 47 | Fase |
| 50 | Betätigungsabschnitt |
| 51 | Innenrippe |
| 52 | Einführungsschräge |
| 53 | Außenrippe |
| 100 | Verschluss |
| 110 | Verschlusskappe |
| 112 | Außenkante der Verschlusskappe |
| 120 | Dichtelement |
| 121 | Dichtabschnitt |
| 122 | Außenkante des Dichtabschnittes |
| 140 | Montageabschnitt |
| 150 | Betätigungsabschnitt |

| S | Setzmaß |
|---|---|
| A | Abstand |
| E | Einspritzpunkt |

**Patentansprüche**

1. Verschluss (1) zum lösbaren Verschließen einer Öffnung (34) am distalen Ende eines zylinderförmigen Behälters (30) für pharmazeutische Präparate, insbesondere einer Spritze, mittels einer Luer-Lock-Befestigung, umfassend

eine zylindrische Verschlusskappe (10) und ein Dichtelement (20), wobei die zylindrische Verschlusskappe (10) einen durchgehenden zylindrischen Hohlraum (14) zur Aufnahme und Halterung des Dichtelements (20) aufweist und wobei

die Verschlusskappe (10) einen Montageabschnitt (40) umfasst, welcher in montierter Position zu dem Behälter hinweist, wobei der Montageabschnitt (40) ein Außengewinde (41) umfasst, welches in montierter Position in die Luer-Lock-Befestigung des Behälters (30) eingreift und eine lösbare Verbindung schafft und wobei das Dichtelement (20) mit einem Dichtabschnitt ausgestaltet ist, welcher zumindest teilweise innerhalb des Montageabschnittes (40) angeordnet ist und welcher in montierter Position die Öffnung umschließt, wobei die Außenkante des Dichtelements (20) gegenüber der Außenkante des Montageabschnittes (40) einen Rückversatz aufweist, **dadurch gekennzeichnet, dass** auf der Flanke des Gewindegangs zumindest ein Erhebungspaar umfassend zwei gegenüberliegend angeordnete Erhebungen vorgesehen ist, derart, dass in montierter Position die Erhebungspunkte dann an der Gewindeflanke der Luer-Lock-Befestigung anliegen und leicht gestaucht oder deformiert werden, so dass beim Öffnen des Verschlusses (1) zu Beginn eine höhere Kraftkomponente aufgewendet werden muss, um das Lösen zu bewirken, wobei, wenn ein Schwellwert überschritten wird, die einzubringende Kraftkomponente kontinuierlich absinkt.

2. Verschluss (1) nach dem vorstehenden Anspruch, **dadurch gekennzeichnet, dass** der Rückversatz der Außenkante des Dichtelements (20) gegenüber der Außenkante des Montageabschnittes (40) wenigstens 0,5 mm, bevorzugt wenigstens 1 mm und besonders bevorzugt wenigstens 1,5 mm beträgt.

3. Verschluss (1) nach einem der vorstehenden Ansprüche, **dadurch gekennzeichnet, dass** die Verschlusskappe (10) ferner einen dem Montageabschnitt (40) gegenüberliegend angeordneten Betätigungsabschnitt (50) umfasst, wobei das Dichtelement (20) zumindest teilweise innerhalb des Betätigungsabschnittes (50) angeordnet ist, und wobei die Außenkante des Dichtelements (20) gegenüber der Außenkante des Betätigungsabschnittes (50) einen Rückversatz aufweist, welcher vorzugsweise wenigstens 0,5 mm, bevorzugt wenigstens 1 mm und besonders bevorzugt wenigstens 1,5 mm beträgt.

4. Verschluss (1) nach einem der vorstehenden Ansprüche, **dadurch gekennzeichnet, dass** das Verhältnis der Länge der Dichtelements (20) zur Länge der Verschlusskappe (10) höchstens 95 %, bevorzugt höchstens 90 % und besonders bevorzugt höchstens 85 % beträgt.

5. Verschluss (1) nach einem der vorstehenden Ansprüche, **dadurch gekennzeichnet, dass** das Dichtelement (20) ein elastomeres Material umfasst.

6. Verschluss (1) nach einem der vorstehenden Ansprüche, **dadurch gekennzeichnet, dass** das Außengewinde (41) als Trapezgewinde ausgebildet ist, vorzugsweise mit einer anderen Form der Gewindeflanke, bevorzugt mit einer Außen- bzw. Kammbreite von 0,6 mm und einer Kernbreite von 1,2 mm.

7. Verschluss (1) nach einem der vorstehenden Ansprüche, **dadurch gekennzeichnet, dass** auf der Flanke des Gewindegangs zwei Erhebungspaare und bevorzugt vier Erhebungspaare vorgesehen sind.

8. Verschluss (1) nach einem der vorstehenden Ansprüche, **dadurch gekennzeichnet, dass** eine axiale Aufwölbung (44) des neben dem Gewindeeingang (46) liegenden Abschnittes des Gewindegangs vorgesehen ist.

9. Verschluss (1) nach einem der vorstehenden Ansprüche, **dadurch gekennzeichnet, dass** der Gewindegang eine Fase (47) umfasst.

10. Verschluss (1) nach einem der vorstehenden Ansprüche, umfassend eine zylindrische Verschlusskappe (10) mit einer durchgehenden Öffnung, wobei die Wandstärke der Verschlusskappe an der dicksten Stelle höchstens 1,5 mm beträgt und/oder die Wandstärke schwankt auf die Länge der Verschlusskappe gesehen um nicht mehr als 0,5 mm, bevorzugt um nicht mehr als 0,3 mm.

11. Verschluss (1) nach einem der vorstehenden Ansprüche, **dadurch gekennzeichnet, dass** der Einspritzpunkt auf einer Position auf der Mantelfläche des Betätigungsabschnittes (50) der Verschlusskappe (10) axial beabstandet von einer Außenrippe (53) angeordnet ist.

12. Verschluss (1) nach einem der vorstehenden Ansprüche, **dadurch gekennzeichnet, dass** der axiale Abstand zwischen einem äußeren Anschlag und einem inneren Anschlag der Verschlusskappe 2,85 mm mit einer Toleranz von +/- 0,2 mm, bevorzugt von +/- 0,1 mm und besonders bevorzugt von +/- 0,05 mm beträgt.

13. Verschluss (1) nach einem der vorstehenden Ansprüche, **dadurch gekennzeichnet, dass** die durchgehende Öffnung (14) der Verschlusskappe (10) zumindest zwei längliche, in ihrer Hauptausrichtung axial angeordnete und nach Innen hervorstehende Innenrippen (51) auf der Innenwandung aufweist, vorzugsweise im Bereich des Betätigungsabschnittes (50), wobei die Innenrippen (51) eine Breite von weniger als 1 mm, bevorzugt von weniger als 0,9 mm und besonders bevorzugt von weniger als 0,8 mm aufweisen.

14. Verschluss (1) nach dem vorstehenden Anspruch, **dadurch gekennzeichnet, dass** die Breite oder der Querschnitt der Innenrippe (51) über einen Großteil der Länge der Innenrippe (51) konstant ist, insbesondere über wenigstens 50 % der Länge der Innenrippe (51), bevorzugt über eine Länge von wenigstens 60 % und besonders bevorzugt von wenigstens 65 %.

15. Verschluss (1) nach einem der vorstehenden Ansprüche, **dadurch gekennzeichnet, dass** der Betätigungsabschnitt (50) zumindest zwei längliche, in ihrer Hauptausrichtung axial angeordnete und radial nach Außen von der Mantelfläche hervorstehende Außenrippen (53) umfasst, welche vorzugsweise eine Länge von wenigstens 4,5 mm, bevorzugt von wenigstens 5,0 mm und besonders bevorzugt 5,3 mm aufweisen.

16. Verschluss (1) nach einem der vorstehenden Ansprüche, **dadurch gekennzeichnet, dass** der Verschluss (1) einen farbstabilen Kunststoff umfasst.

17. Verschluss (1) nach dem vorstehenden Anspruch, **dadurch gekennzeichnet, dass** die Verschlussfarbe graugrün ist und nach dem RAL-Farbsystem dem Farbton RAL 7009 entspricht, wobei der Verschluss (1) vorzugsweise farbstabil ist gegenüber Einwirkungen infolge einer Behandlung mit einer der folgenden Sterilisationsmethoden: Heißluft, Heißdampf bei bis zu 134 °C für eine Zeitdauer von 10 Minuten, GammaStrahlen oder Röntgen-Strahlen mit einer Energie-Dosis im Bereich von 25-40 kGy oder einer Kombination dieser Methoden.

18. Verschluss (1) nach einem der beiden vorstehenden Ansprüche, **dadurch gekennzeichnet, dass** das Material für den Verschluss (1) die folgenden Pigmente als farbgebende Elemente umfasst:

    - Titandioxid Kronos® 2233
    - Ruß PRINTEX® F 85
    - 6975 Titanorange
    - VYNAMON ® Green 600734.

19. Verfahren zum kontaminationsfreien lösbaren Verschließen eines distalen Endes eines zylinderförmigen Behälters (30) für pharmazeutische Präparate, insbesondere einer Spritze, mittels einer Luer-Lock-Befestigung, mit einem Verschluss (1) nach einem der vorstehenden Ansprüche.

20. Verfahren zum Verschließen eines Behälters (30) nach vorstehendem Anspruch, **dadurch gekennzeichnet, dass** das Anziehdrehmoment und/oder das Öffnungsdrehmoment eine geringe Streuung von +/- 1,0 N*cm, bevorzugt +/- 0,5 N*cm, zum jeweils geforderten Drehmoment aufweisen.

21. Verfahren zur Herstellung eines Verschlusses, insbesondere einem Verschluss (1) nach einem der vorstehenden Ansprüche 1-18, **dadurch gekennzeichnet, dass** der Verschluss (1) ein Betätigungselement mit zumindest zwei Außenrippen (53) umfasst und mittels Spritzgussverfahren hergestellt wird, wobei ein Einspritzpunkt gewählt wird, welcher im Bereich des Betätigungsabschnitts (50) liegt, vorzugsweise an einer Position auf der außenseitigen Mantelfläche des Betätigungsabschnittes (50) axial beabstandet von einer Außenrippe (53).

## Claims

1. A closure (1) for releasably closing an opening (34) at the distal end of a cylindrical container (30) for pharmaceutical preparations, in particular a syringe, by Luer lock fastening means, comprising

    a cylindrical closure cap (10) and a sealing element (20), wherein the cylindrical closure cap (10) has a continuous cylindrical cavity (14) for receiving and holding the sealing element (20), and wherein the closure cap (10) comprises a mounting section (40) which, in the mounted position, faces the container, wherein the mounting section (40) has an external thread (41) which, in the mounted position, engages in the Luer lock fastening means of the container (30) and creates a releasable connection; and wherein the sealing element (20) is designed with a sealing section which is arranged at least partially within the mounting section (40) and which, in the mounted position, surrounds the opening, wherein the outer edge of the sealing element (20) is set back relative to the outer edge of the mounting section (40);
    **characterized in that** on the flank of the thread turn, at least one pair of protrusions comprising two oppositely arranged protrusions is provided in such a way that, in the mounted position, the protruding points engage on the thread flank of the Luer lock fastening means and are slightly compressed or deformed, so that when opening the closure (1), a higher force component must be applied initially in order to effect the release, wherein the force component to be applied continuously decreases once a threshold value has been exceeded.

2. The closure (1) according to the preceding claim, **characterized in that** the setback of the outer edge of the sealing element (20) relative to the outer edge of the mounting section (40) is at least 0.5 mm, preferably at least 1 mm and most preferably at least 1.5 mm.

3. The closure (1) according to any one of the preceding claims, **characterized in that** the closure cap (10) furthermore comprises an actuation section (50) arranged opposite the mounting section (40), wherein the sealing element (20) is arranged at least partially inside the actuation section (50); and wherein the outer edge of the sealing element (20) is set back relative to the outer edge of the actuation section (50) by preferably at least 0.5 mm, more preferably at least 1 mm and most preferably at least 1.5 mm.

4. The closure (1) according to any one of the preceding claims, **characterized in that** the ratio of the length of the sealing element (20) to the length of the closure cap (10) is at most 95 %, preferably at most 90 % and most preferably at most 85 %.

5. The closure (1) according to any one of the preceding claims, **characterized in that** the sealing element (20) comprises an elastomeric material.

6. The closure (1) according to any one of the preceding claims, **characterized in that** the external thread (41) is in the form of a trapezoidal thread, preferably with a modified shape of the thread flank, preferably with an outer width or crest width of 0.6 mm and a root width of 1.2 mm.

7. The closure (1) according to any one of the preceding claims, **characterized in that** the flank of the thread turn is provided with two pairs of protrusions and preferably with four pairs of protrusions.

8. The closure (1) according to any one of the preceding claims, **characterized in that** an axial bulge (44) is provided on the portion of the thread turn adjacent to the thread entry (46).

9. The closure (1) according to any one of the preceding claims, **characterized in that** the thread turn has a chamfer (47).

10. The closure (1) according to any one of the preceding claims, comprising a cylindrical closure cap (10) with a through opening, wherein the wall thickness of the closure cap is at most 1.5 mm at the thickest point, and/or wherein the wall thickness varies over the length of the closure cap by not more than 0.5 mm, preferably by not more than 0.3 mm.

11. The closure (1) according to any one of the preceding claims, **characterized in that** the injection point is provided at a position on the lateral surface of the actuation section (50) of the closure cap (10) axially spaced apart from an outer rib (53).

12. The closure (1) according to any one of the preceding claims, **characterized in that** the axial spacing between an outer stop and an inner stop of the closure cap is 2.85 mm with a tolerance of $\pm0.2$ mm, preferably $\pm0.1$ mm and most preferably $\pm0.05$ mm.

13. The closure (1) according to any one of the preceding claims, **characterized in that** the through opening (14) of the closure cap (10) has at least two elongated inner ribs (51) on the inner wall, which are arranged axially in its main orientation and project inwards, preferably in the area of the actuation section (50), wherein said inner ribs (51) have a width of less than 1 mm, preferably of less than 0.9 mm and most preferably of less than 0.8 mm.

14. The closure (1) according to the preceding claim, **characterized in that** the width or cross section of the inner rib (51) is consistent over a large part of the length of the inner rib (51), in particular over at least 50 % of the length of the inner rib (51), preferably over at least 60 % of the length and most preferably of at least 65 %.

15. The closure (1) according to any one of the preceding claims, **characterized in that** the actuation section (50) comprises at least two elongated outer ribs (53), which are arranged axially in their main orientation and project radially outwards from the lateral surface, which preferably have a length of at least 4.5 mm, more preferably of at least 5.0 mm and most preferably of 5.3 mm.

16. The closure (1) according to any one of the preceding claims, **characterized in that** the closure (1) comprises a colour-stable plastics material.

17. The closure (1) according to the preceding claim, **characterized in that** the colour of the closure is grey-green and corresponds to the colour RAL 7009 according to the RAL colour system, wherein the closure (1) is preferably colour-stable against effects resulting from treatment with any of the following sterilization methods: hot air, hot steam at up to 134 °C over a duration of 10 minutes, gamma rays or X-rays with an energy dose in the range of 25 - 40 kGy or a combination of these methods.

**18.** The closure (1) according to any one of the two preceding claims, **characterized in that** the material for the closure (1) comprises the following pigments as colour-imparting elements:

- Titanium dioxide Kronos® 2233
- Carbon black PRINTEX® F 85
- 6975 Titanium Orange
- VYNAMON® Green 600734.

**19.** A method for sealing a distal end of a cylindrical container (30) for pharmaceutical preparations, in particular a syringe, in a contamination-free, releasable manner by Luer lock fastening means using a closure (1) according to any one of the preceding claims.

**20.** The method for sealing a container (30) according to the preceding claim, **characterized in that** the tightening torque and/or the opening torque exhibit a small variation of ±1.0 N·cm, preferably ±0.5 N·cm, relative to the respective nominal torque.

**21.** A method for producing a closure, in particular a closure (1) according to any one of the preceding claims 1 - 18, **characterized in that** the closure (1) comprises an actuation element with at least two outer ribs (53) and is produced by an injection moulding process, wherein an injection point is selected which lies in the area of the actuation section (50), preferably at a position on the outer lateral surface of the actuation section (50) axially spaced apart from an outer rib (53).

**Revendications**

**1.** Fermeture (1) pour fermer de manière libérable une ouverture (34) à l'extrémité distale d'un récipient cylindrique (30) pour préparations pharmaceutiques, en particulier une seringue, au moyen d'une fixation Luer-Lock, comprenant un capuchon de fermeture (10) cylindrique et un élément d'étanchéité (20), dans laquelle le capuchon de fermeture (10) cylindrique présente une cavité cylindrique continue (14) pour recevoir et maintenir l'élément d'étanchéité (20) et dans laquelle le capuchon de fermeture (10) comprend une section de montage (40) laquelle, en position montée, est tournée vers le récipient, dans laquelle la section de montage (40) comprend un filetage mâle (41) lequel, en position montée, s'insère dans la fixation Luer-Lock du récipient (30) et crée une liaison libérable

et dans laquelle l'élément d'étanchéité (20) est conçu avec une section d'étanchéité, laquelle est disposée au moins partiellement à l'intérieur de la section de montage (40) et laquelle entoure l'ouverture en position montée, dans laquelle le bord extérieur de l'élément d'étanchéité (20) présente un décalage vers l'arrière par rapport au bord extérieur de la section de montage (40), **caractérisée en ce qu'**au moins une paire de bossages comprenant deux bossages disposés en vis-à-vis est prévue sur le flanc du pas de filetage, de sorte, que, en position montée, les points de bossage reposent alors contre le flanc de filetage de la fixation Luer-Lock et sont légèrement comprimés ou déformés, de sorte qu'à l'ouverture de la fermeture (1), une composante de force plus élevée doit être appliquée au début pour provoquer la libération, dans laquelle, si une valeur seuil est dépassée, la composante de force à introduire baisse continuellement.

**2.** Fermeture (1) selon la revendication précédente, **caractérisée en ce que** le décalage vers l'arrière du bord extérieur de l'élément d'étanchéité (20) par rapport au bord extérieur de la section de montage (40) est d'au moins 0,5 mm, de préférence d'au moins 1 mm et de manière particulièrement préférée d'au moins 1,5 mm.

**3.** Fermeture (1) selon l'une quelconque des revendications précédentes, **caractérisée en ce que** le capuchon de fermeture (10) comprend en outre une section d'actionnement (50) disposée en vis-à-vis de la section de montage (40), dans laquelle l'élément d'étanchéité (20) est disposé au moins partiellement à l'intérieur de la section d'actionnement (50), et dans laquelle le bord extérieur de l'élément d'étanchéité (20) présente par rapport au bord extérieur de la section d'actionnement (50) un décalage vers l'arrière, lequel est de préférence d'au moins 0,5 mm, de préférence d'au moins 1 mm et de manière particulièrement préférée d'au moins 1,5 mm.

**4.** Fermeture (1) selon l'une quelconque des revendications précédentes, **caractérisée en ce que** le rapport de la longueur de l'élément d'étanchéité (20) à la longueur du capuchon de fermeture (10) est de 95 % maximum, de préférence de 90 % maximum et de manière particulièrement préférée de 85 % maximum.

**5.** Fermeture (1) selon l'une quelconque des revendications précédentes, **caractérisée en ce que** l'élément d'étanchéité (20) présente un matériau élastomère.

**6.** Fermeture (1) selon l'une quelconque des revendications précédentes, **caractérisée en ce que** le filetage extérieur (41) est réalisé en tant que filetage trapézoïdal, de préférence avec une autre forme du

EP 3 569 272 B1

flanc de filetage, de préférence avec une largeur extérieure ou de peigne de 0,6 mm et une largeur de noyau de 1,2 mm.

7. Fermeture (1) selon l'une quelconque des revendications précédentes, **caractérisée en ce que** deux paires de bossages et de préférence quatre paires de bossages sont prévues sur le flanc du pas de filetage.

8. Fermeture (1) selon l'une quelconque des revendications précédentes, **caractérisée en ce qu'**un bombement axial (44) de la section du pas de filetage située à côté de l'entrée de filetage (46) est prévu.

9. Fermeture (1) selon l'une quelconque des revendications précédentes, **caractérisée en ce que** le pas de filetage comprend un chanfrein (47).

10. Fermeture (1) selon l'une quelconque des revendications précédentes, comprenant un capuchon de fermeture (10) cylindrique avec une ouverture traversante, dans laquelle l'épaisseur de paroi du capuchon de fermeture à l'endroit le plus épais est de 1,5 mm au maximum et/ou l'épaisseur de paroi varie vu sur la longueur du capuchon de fermeture de pas plus de 0,5 mm, de préférence de pas plus de 0,3 mm.

11. Fermeture (1) selon l'une quelconque des revendications précédentes, **caractérisée en ce que** le point d'injection est disposé à une position sur la surface d'enveloppe de la section d'actionnement (50) du capuchon de fermeture (10) espacée axialement d'une nervure extérieure (53).

12. Fermeture (1) selon l'une quelconque des revendications précédentes, **caractérisée en ce que** la distance axiale entre une butée extérieure et une butée intérieure du capuchon de fermeture est de 2,85 mm avec une tolérance de +/- 0,2 mm, de préférence de +/- 0,1 mm et de manière particulièrement préférée de +/- 0,05 mm.

13. Fermeture (1) selon l'une quelconque des revendications précédentes, **caractérisée en ce que** l'ouverture traversante (14) du capuchon de fermeture (10) présente sur la paroi intérieure au moins deux nervures intérieures (51) allongées, disposées axialement dans leur orientation principale et faisant saillie vers l'intérieur, de préférence dans la zone de la section d'actionnement (50), dans laquelle les nervures intérieures (51) présentent une largeur inférieure à 1 mm, de préférence inférieure à 0,9 mm et de manière particulièrement préférée inférieure à 0,8 mm.

14. Fermeture (1) selon la revendication précédente, **caractérisée en ce que** la largeur ou la section de la nervure intérieure (51) est constante sur une grande partie de la longueur de la nervure intérieure (51), en particulier sur au moins 50 % de la longueur de la nervure intérieure (51), de préférence sur une longueur d'au moins 60 % et de manière particulièrement préférée d'au moins 65 %.

15. Fermeture (1) selon l'une quelconque des revendications précédentes, **caractérisée en ce que** la section d'actionnement (50) comprend au moins deux nervures extérieures (53) allongées, disposées axialement dans leur orientation principale et faisant saillie radialement vers l'extérieur par rapport à la surface d'enveloppe, lesquelles présentent de préférence une longueur d'au moins 4,5 mm, de préférence d'au moins 5,0 mm et de manière particulièrement préférée de 5,3 mm.

16. Fermeture (1) selon l'une quelconque des revendications précédentes, **caractérisée en ce que** la fermeture (1) comprend un plastique stable en couleur.

17. Fermeture (1) selon la revendication précédente, **caractérisée en ce que** la couleur de fermeture est gris-vert et correspond selon le système de couleurs RAL à la teinte RAL 7009, dans laquelle la fermeture (1) est de préférence stable en couleur face aux effets résultant d'un traitement avec l'une des méthodes de stérilisation suivantes : air chaud, vapeur chaude jusqu'à 134 °C pendant 10 minutes, rayons gamma ou rayons X avec une dose d'énergie dans la plage de 25-40 kGy ou une combinaison de ces méthodes.

18. Fermeture (1) selon l'une des deux revendications précédentes, **caractérisée en ce que** le matériau pour la fermeture (1) comprend les pigments suivants en tant qu'éléments colorants :

- dioxyde de titane Kronos® 2233
- noir de carbone PRINTEX® F 85
- 6975 orange titane
- VYNAMON® vert 600734.

19. Procédé de fermeture libérable sans contamination d'une extrémité distale d'un récipient cylindrique (30) pour préparations pharmaceutiques, en particulier une seringue, au moyen d'une fixation Luer-Lock, avec une fermeture (1) selon l'une quelconque des revendications précédentes.

20. Procédé de fermeture d'un récipient (30) selon la revendication précédente, **caractérisé en ce que** le couple de serrage et/ou le couple d'ouverture présentent une faible dispersion de +/- 1,0 N*cm, de préférence +/-0,5 N*cm, par rapport au couple requis respectivement.

21. Procédé de fabrication d'une fermeture, en particulier d'une fermeture (1) selon l'une quelconque des revendications 1 à 18 précédentes, **caractérisé en ce que** la fermeture (1) comprend un élément d'actionnement avec au moins deux nervures extérieures (53) et est réalisée au moyen d'un procédé de moulage par injection, dans lequel un point d'injection, lequel se trouve dans la zone de la section d'actionnement (50), est choisi de préférence à une position sur la surface d'enveloppe extérieure de la section d'actionnement (50) espacée axialement d'une nervure extérieure (53).

Stand der Technik

Fig. 1

Fig. 2

EP 3 569 272 B1

Fig. 3

Drehmoment (N*cm)

Drehmoment (N*cm)

Winkel (°)

Winkel (°)

Stand der Technik

Fig. 4

Fig. 5

EP 3 569 272 B1

Fig. 6

Fig. 9

EP 3 569 272 B1

Fig. 8

Fig. 7

24

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5624402 A
- US 6196998 B1
- EP 1600190 B1
- WO 2012116791 A1
- EP 1419819 A1
- WO 2013149821 A1
- EP 3015130 A1
- US 9821152 B1